(19) Europäisches Patentamt
European Patent Office
Office européen des brevets

(11) **EP 4 386 761 A2**

(12) **EUROPEAN PATENT APPLICATION**

(43) Date of publication:
**19.06.2024 Bulletin 2024/25**

(21) Application number: **24157818.6**

(22) Date of filing: **08.11.2017**

(51) International Patent Classification (IPC):
***G16B 25/00*** *(2019.01)*

(52) Cooperative Patent Classification (CPC):
(C-Sets available)
**C12Q 1/6841; G16B 25/00** (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.11.2016 US 201662419033 P**

(62) Document number(s) of the earlier application(s) in
accordance with Art. 76 EPC:
**17869122.6 / 3 539 036**

(71) Applicant: **President and Fellows of Harvard
College
Cambridge, MA 02138 (US)**

(72) Inventors:
• **ZHUANG, Xiaowei
Lexington, MA 02421 (US)**
• **MOFFITT, Jeffrey R.
Somerville, MA 02143 (US)**
• **HAO, Junjie George
Cambridge, MA 02140 (US)**
• **LU, Tian
Cambridge, MA 02138 (US)**

(74) Representative: **Hoffmann Eitle
Patent- und Rechtsanwälte PartmbB
Arabellastraße 30
81925 München (DE)**

Remarks:
•The complete document including Reference
Table(s) and the Sequence Listing(s) can be
downloaded from the EPO website
•This application was filed on 15.02.2024 as a
divisional application to the application mentioned
under INID code 62.
•Claims filed after the date of filing of the application
/ after the date of receipt of the divisional application
(Rule 68(4) EPC).

(54) **MATRIX IMPRINTING AND CLEARING**

(57) The present invention generally relates to systems and methods for imaging or determining nucleic acids or other desired targets, for instance, within cells or tissues. In one aspect, a sample is exposed to a plurality of nucleic acid probes that are determined within the sample. In some cases, however, background fluorescence or off-target binding may make it more difficult to determine properly bound nucleic acid probes. Accordingly, other components of the samples that may be contributing to the background, such as proteins, lipids, and/or other non-targets, may be "cleared" from the sample to improve determination. However, in certain embodiments, nucleic acids or other desired targets may be prevented from also being cleared, e.g., using polymers or gels within the sample. Other aspects are generally directed to compositions or kits involving such systems, methods of using such systems, or the like.

Embed → Digest →

〜〜〜 mRNA    ⊔ Encoding probe    ⊙ Anchor probe

FIG. 1B

EP 4 386 761 A2

(52) Cooperative Patent Classification (CPC): (Cont.)

C-Sets
**C12Q 1/6841, C12Q 2537/143**

**Description**

**RELATED APPLICATIONS**

This application claims the benefit of U.S. Provisional Patent Application Serial No. 62/419,033, filed November 8, 2016, entitled "Matrix Imprinting and Clearing," by Zhuang, et al., incorporated herein by reference in its entirety.

**GOVERNMENT FUNDING**

This invention was made with government support under Grant Nos. R01 MH113094 and R01 MH111502 awarded by the NIH. The government has certain rights in the invention.

**FIELD**

The present invention generally relates to systems and methods for imaging or determining nucleic acids or other desired targets, for instance, within cells.

**BACKGROUND**

Highly multiplexed single-molecule fluorescence *in situ* hybridization (smFISH) has emerged as a promising approach to spatially resolved single-cell transcriptomics due to its ability to directly image and profile numerous RNA species in their native cellular context. However, background-from factors such as off-target binding of FISH probes or cellular autofluorescence-can become limiting in a number of important applications, such as imaging shorter RNAs, increasing the degree of multiplexing, and imaging in tissue samples. Accordingly, improvements in such techniques are needed.

**SUMMARY**

The present invention generally relates to systems and methods for imaging or determining nucleic acids, for instance, within cells. The subject matter of the present invention involves, in some cases, interrelated products, alternative solutions to a particular problem, and/or a plurality of different uses of one or more systems and/or articles.

In one set of embodiments, the method comprises exposing a sample to a plurality of nucleic acid probes, polymerizing a gel within the sample, anchoring a target to the gel, clearing non-targets from the sample, and determining the targets within the gel by determining binding of the nucleic acid probes by imaging.

The method, in another set of embodiments, includes exposing a sample to a plurality of nucleic acid probes, polymerizing a gel within the sample, anchoring a target to the gel, reducing background fluorescence within the sample, and imaging the nucleic acid probes.

In yet another set of embodiments, the method includes acts of exposing a sample to a plurality of MERFISH nucleic acid probes, exposing a sample to a plurality of anchor nucleic acid probes, embedding at least a portion of the sample within a polyacrylamide gel, immobilizing at least some of the anchor nucleic acid probes to the polyacrylamide gel, clearing proteins and/or lipids and/or DNA and/or extracellular matrix and/or RNA molecules from the sample, and determining binding of the MERFISH nucleic acid probes by imaging the polyacrylamide gel. In some embodiments, the method includes acts of exposing a sample to a plurality of nucleic acid probes, exposing a sample to a plurality of anchor nucleic acid probes, embedding at least a portion of the sample within a polyacrylamide gel, immobilizing at least some of the anchor nucleic acid probes to the polyacrylamide gel, clearing proteins and/or lipids and/or DNA and/or extracellular matrix and/or RNA molecules from the sample, and determining binding of the nucleic acid probes by imaging the polyacrylamide gel.

According to still another set of embodiments, the method includes embedding at least a portion of a sample within a matrix, immobilizing targets to the matrix, clearing non-targets from the matrix, and imaging the targets within the matrix.

In another aspect, the present invention encompasses methods of making one or more of the embodiments described herein. In still another aspect, the present invention encompasses methods of using one or more of the embodiments described herein.

Other advantages and novel features of the present invention will become apparent from the following detailed description of various non-limiting embodiments of the invention when considered in conjunction with the accompanying figures.

## BRIEF DESCRIPTION OF THE DRAWINGS

Non-limiting embodiments of the present invention will be described by way of example with reference to the accompanying figures, which are schematic and are not intended to be drawn to scale. In the figures, each identical or nearly identical component illustrated is typically represented by a single numeral. For purposes of clarity, not every component is labeled in every figure, nor is every component of each embodiment of the invention shown where illustration is not necessary to allow those of ordinary skill in the art to understand the invention. In the figures:

Figs. 1A-1C illustrate a reduction of background in accordance with one embodiment of the invention;
Figs. 2A-2D illustrate a reduction of background without loss of RNA, in another embodiment of the invention;
Figs. 3A-3E illustrate a reduction of background in multiple color imaging, in yet another embodiment of the invention;
Figs. 4A-4G illustrate a reduction of background in tissue, in still another embodiment of the invention;
Figs. 5A-5C illustrates MERFISH, in accordance with one embodiment of the invention;
Fig. 6 illustrates off-target binding, in accordance with another embodiment of the invention;
Figs. 7A-7D illustrate clearance using protease digestion and detergent, in yet another embodiment;
Figs. 8A-8B shows that clearance does not reduce probe binding, in still another embodiment of the invention;
Figs. 9A-9B illustrate a reduction in bias in the detection of low abundance RNAs, in yet another embodiment of the invention; and
Fig. 10 illustrates reproducibility, in certain embodiments of the invention.

## DETAILED DESCRIPTION

The present invention generally relates to systems and methods for imaging or determining nucleic acids or other desired targets, for instance, within cells or tissues. In one aspect, a sample is exposed to a plurality of nucleic acid probes that are determined within the sample. In some cases, however, background fluorescence or off-target binding may make it more difficult to determine properly bound nucleic acid probes. Accordingly, other components of the samples that may be contributing to the background, such as proteins, lipids, and/or other non-targets, may be "cleared" from the sample to improve determination. However, in certain embodiments, nucleic acids or other desired targets may be prevented from also being cleared, e.g., using polymers or gels within the sample. Other aspects are generally directed to compositions or kits involving such systems, methods of using such systems, or the like.

Thus, in one aspect, the present invention is generally directed to systems and methods for preventing nucleic acids, or other desired targets, within a sample from being cleared, e.g., by immobilizing the nucleic acids or other desired targets. In some cases, the nucleic acids or other targets may thus be imaged or otherwise determined within the sample. For instance, a plurality of nucleic acid probes can be applied to a sample, and their binding within the sample determined, e.g., using fluorescence, to determine locations of the nucleic acid probes within the sample. In addition, in some cases, a plurality of nucleic acid probes may be successively applied to the sample. In other embodiments, other targets can be determined within a sample, e.g., in addition to and/or instead of nucleic acids. Accordingly, it should be understood that nucleic acids are presented here for purposes of clarity, but in other embodiments, other targets may be determined.

Without wishing to be bound by any theory, it is believed that certain components such as proteins and lipids, unbound or irrelevant nucleic acids, fluorescent components (bleached or unbleached), or the like may create problems in imaging or analysis, e.g., due to autofluorescence, components that quench fluorescent molecules, off-target binding, or other phenomena. For example, it is believed that nucleic acid probes may not bind to a proper target within a sample, and instead may bind "off-target" to other cellular components, including but not limited to proteins, lipids, RNA, DNA, etc. Similarly, probes targeting one DNA or RNA molecule may bind "off-target" to the wrong DNA or RNA molecule. These interactions could be driven, for example, by imperfect base pairing, charge-charge interactions, or other molecular interactions.

Accordingly, in certain embodiments, a polymer or gel may be applied to a sample to immobilize desired nucleic acid molecules (or other desired targets), while the components to which nucleic acid probes bind off-target can be cleared from the sample, e.g. by removal and/or degradation techniques. This may reduce the amount of probes that bind off-target, facilitating imaging or other analysis of the sample. Other components, such as proteins and lipids, may be cleared from the sample, e.g., by removal and/or degradation techniques. This may reduce the amount of background, facilitating imaging or other analysis of the sample.

For example, in one set of embodiments, a sample is exposed to a plurality of oligonucleotide probes. The sample can be a biological sample, e.g., cells or tissue. The probes may be, for example, smFISH or MERFISH probes, and may be substantially complementary to mRNA or other RNAs, for example, for transcriptome analyses. The probes may also include signaling entities, e.g., fluorescent signaling entities, for imaging and/or analysis of the sample. In addition, in some cases, anchor probes may also be included, which may be used to immobilize the probes to a polymer or gel, as discussed below. In some cases, for example, the anchor probes may contain portions comprising thymine residues (e.g., for binding to a poly-A tail of an mRNA). In addition, in some embodiments, the anchor probes may contain sequences complementary to the desired nucleic acid species, e.g., binding to them via base pairing. Anchor probes, in some embodiments, may contain portions able to polymerize with a gel or protein. After exposure to the sample, the nucleic acid probes may associate with RNA, DNA, or other components within the sample.

In some embodiments, the sample is embedded within a matrix that immobilizes nucleic acids, e.g., before application of the nucleic acid probes. For instance, the matrix may comprise a gel or a polymer, such as polyacrylamide. Thus, for example, acrylamide and a suitable cross-linker (e.g., N,N'-methylenebisacrylamide) can be added to the sample and polymerized to form a gel. The anchor probes, if present, may include a portion able to polymerize with the gel (e.g., an acrydite moiety) during the polymerization process, and nucleic acids (e.g., mRNAs containing poly-A tails) may then be able to associate with the anchor portion. In such fashion, the mRNAs may be immobilized to the polyacrylamide gel. As another example, DNA and/or RNA molecules may be immobilized to the polyacrylamide gel using anchor probes having substantially complementary portions to the DNA or RNA. As yet another example, DNA and/or RNA molecules may be physically tangled within the polyacrylamide gel, e.g., due to their length, to immobilize them to the polyacrylamide gel.

After immobilization, other components may be "cleared" from the sample. Such clearance may include removal (e.g., physical removal) from the sample, and/or degradation, such that they are no longer as prominent within the background. Degradation may include, for example, chemical degradation, enzymatic degradation, or the like. For instance, proteins within the sample may be "flushed" from the gel by exposing the gel to a suitable fluid, e.g., a buffer solution. Components such as enzymes (e.g., proteinases, digestive enzymes, etc.), denaturants (e.g., guanidine HCl), etc. may be applied to the proteins to digest the proteins into smaller fragments, individual amino acids, etc., which may be easier to remove from the sample, or may be small or dim enough that their presence can be ignored. Similarly, lipids may be cleared using surfactants such as Triton X-100 or SDS, and ions may be cleared using EDTA, or the like. In some cases, these may be combined together. As mentioned, it is believed that such components may increase background, e.g., when using fluorescence or other microscopy techniques, and thus, removal of such components should decrease the background. However, it should be noted that nucleic acids immobilized within the polymer or gel may not be cleared or removed, and thus remain available for analysis.

The above discussion is a non-limiting example of one embodiment of the present invention. However, other embodiments are also possible. Accordingly, more generally, various aspects of the invention are directed to various systems and methods for imaging or determining nucleic acids or other desired targets, for instance, within cells, tissues or other samples. For example, in certain embodiments, a desired target is immobilized within an inert matrix (such as a polymer or gel), while other components are "cleared," e.g., via degradation and/or physical removal.

The sample may be any suitable sample, and may be biological. In some cases, the sample contains DNA and/or RNA, e.g., that may be determined within the sample. (In other embodiments, other targets within the sample may be determined.) In some cases, the sample may include cells, such as mammalian cells or other types of cells. The sample may contain viruses in some cases. In addition, in some cases, the sample may be a tissue sample, e.g., from a biopsy, artificially grown or cultured, etc.

If nucleic acids are desired to be determined, the nucleic acids may be, for example, DNA, RNA, or other nucleic acids that are present within a cell (or other sample). The nucleic acids may be endogenous to the cell, or added to the cell. For instance, the nucleic acid may be viral, or artificially created. In some cases, the nucleic acid to be determined may be expressed by the cell. The nucleic acid is RNA in some embodiments. The RNA may be coding and/or non-coding RNA. Non-limiting examples of RNA that may be studied within the cell include mRNA, siRNA, rRNA, miRNA, tRNA, lncRNA, snoRNAs, snRNAs, exRNAs, piRNAs, or the like.

In some cases, a significant portion of the nucleic acid within the cell may be studied. For instance, in some cases, enough of the RNA present within a cell may be determined so as to produce a partial or complete transcriptome of the cell. In some cases, at least 4 types of mRNAs are determined within a cell, and in some cases, at least 3, at least 4, at least 7, at least 8, at least 12, at least 14, at least 15, at least 16, at least 22, at least 30, at least 31, at least 32, at least 50, at least 63, at least 64, at least 72, at least 75, at least 100, at least 127, at least 128, at least 140, at least 255, at least 256, at least 500, at least 1,000, at least 1,500, at least 2,000, at least 2,500, at least 3,000, at least 4,000, at least 5,000, at least 7,500, at least 10,000, at least 12,000, at least 15,000, at least 20,000, at least 25,000, at least 30,000, at least 40,000, at least 50,000, at least 75,000, or at least 100,000 types of mRNAs may be determined within a cell.

In some cases, the transcriptome of a cell may be determined. It should be understood that the transcriptome generally encompasses all RNA molecules produced within a cell, not just mRNA. Thus, for instance, the transcriptome may also include rRNA, tRNA, siRNA, etc. In some embodiments, at least 5%, at least 10%, at least 15%, at least 20%, at least 25%, at least 30%, at least 40%, at least 50%, at least 60%, at least 70%, at least 80%, at least 90%, or 100% of the transcriptome of a cell may be determined.

The determination of one or more nucleic acids within the cell or other sample may be qualitative and/or quantitative. In addition, the determination may also be spatial, e.g., the position of the nucleic acid within the cell or other sample may be determined in two or three dimensions. In some embodiments, the positions, number, and/or concentrations of nucleic acids within the cell (or other sample) may be determined.

In some cases, a significant portion of the genome of a cell may be determined. The determined genomic segments may be continuous or interspersed on the genome. For example, in some cases, at least 4 genomic segments are determined within a cell, and in some cases, at least 3, at least 4, at least 7, at least 8, at least 12, at least 14, at least 15, at least 16, at least 22, at least 30, at least 31, at least 32, at least 50, at least 63, at least 64, at least 72, at least 75, at least 100, at least 127, at least 128, at least 140, at least 255, at least 256, at least 500, at least 1,000, at least 1,500, at least 2,000, at least 2,500, at least 3,000, at least 4,000, at least 5,000, at least 7,500, at least 10,000, at least 12,000, at least 15,000, at least 20,000, at least 25,000, at least 30,000, at least 40,000, at least 50,000, at least 75,000, or at least 100,000 genomic segments may be determined within a cell.

In some cases, the entire genome of a cell may be determined. It should be understood that the genome generally encompasses all DNA molecules produced within a cell, not just chromosome DNA. Thus, for instance, the genome may also include, in some cases, mitochondria DNA, chloroplast DNA, plasmid DNA, etc. In some embodiments, at least about 5%, at least about 10%, at least about 15%, at least about 20%, at least about 25%, at least about 30%, at least about 40%, at least about 50%, at least about 60%, at least about 70%, at least about 80%, at least about 90%, or 100% of the genome of a cell may be determined.

However, as discussed, it should be understood that in other embodiments of the invention, other targets may be determined or immobilized, e.g., in addition to and/or instead of nucleic acids. For example, in some embodiments of the invention, the targets to be determined or immobilized may include proteins (e.g., antibodies, enzymes, structural proteins), lipids, carbohydrates, viruses, or the like. In one embodiment, cellular components, such as proteins, can be detected by binding to them proteins, such as antibodies, that are conjugated to oligonucleotide probes which are anchored to the polymer or gel matrix. These components could then be removed, leaving the oligonucleotide probes to be detected via hybridization of additional nucleic acid probes, similar or identical to the detection of cellular nucleic acids. In another embodiment, multiple distinct cellular species could be detected simultaneously within the same sample, even if the original components are removed from the gel or polymer. For example, RNA molecules could be detected via hybridization of nucleic acid probes simultaneously with the detection of proteins via antibody-oligonucleotide conjugates, as described above.

As mentioned, the sample may be immobilized or embedded within a polymer or a gel, partially or completely. In some cases, the sample may be embedded within a relatively large polymer or gel, which can then be sectioned or sliced in some cases to produce smaller portions for analysis, e.g., using various microtomy techniques commonly available to those of ordinary skill in the art. For instance, tissues or organs may be immobilized within a suitable polymer or gel.

A variety of polymers may be used in some embodiments. In some cases, the polymer may be selected to be relatively optically transparent. The polymer may also be one that does not significantly distort during the polymerization process, although in some cases, the polymer may exhibit some distortion. In some cases, the amount of distortion may be determined as a relative change in size that is less than 5, less than 4, less than 3, less than 2, less than 1.5, less than 1.3, or less than 1.2 (i.e., a change in size of 2 means that a sample doubles in linear dimension), or inverses of these (i.e., an inverse change in size of 2 means that a sample halves in linear dimensions).

Examples of suitable polymers include polyacrylamide and agarose. In some cases, the polymer is a gel or a hydrogel. A variety of polymers could be used in various embodiments that involve chemical cross links between gel subunits, including but not limited to acrylic acid, acrylamide, ethylene glycol diacrylate, ethylene glycol dimetharcrylate, poly(ethylene glycol dimethacrylate); and/or hydrophobic or hydrogen bonding interactions, such as poly(*N*-isopropyl acrylamide), methyl cellulose, (ethylene oxide)-(propylene oxide)-(ethylene oxide terpolymers, sodium alginate, poly(vinyl alcohol), alignate, chitosan, gum Arabic, gelatin, and agarose.

In one set of embodiments, anchor probes may be used during the polymerization process. The anchor probes may include a portion that is able to polymerize with the polymer during the polymerization process, and is able to immobilize a target, e.g., chemically and/or physically. For example, in the case of polyacrylamide, the anchor probe may include an acrydite portion that can polymerize and become incorporated into the polymer.

The anchor probe may also contain a portion that can interact with and bind to nucleic acid molecules, or other molecules in which immobilization is desired, e.g., proteins or lipids, other desired targets, etc. The immobilization may be covalent or non-covalent. For example, to immobilize a target nucleic acid, the anchor probe may comprise a nucleic acid comprising an acrydite portion (e.g., at the 5' end, the 3' end, an internal base, etc.) and a nucleic acid sequence substantially complementary to at least a portion of the target nucleic acid. For instance, the nucleic acid may be complementary to at least 5, 6, 7, 8, 9, 10, 11, 12, 13, 14, 15, 16, 17, 18, 19, or 20 or more nucleotides of the nucleic acid. In some cases the complementarity may be exact (Watson-Crick complementarity), or there may be 1, 2, or more mismatches. In some cases, the anchor probe can be configured to immobilize mRNA, e.g., in the case of transcriptome analysis. For instance, in one set of embodiments, the anchor probe may contain a plurality of thymine nucleotides, e.g., sequentially, for binding to the poly-A tail of an mRNA. Thus, for example, the anchor probe can have at least 5, 6, 7, 8, 9, 10, 11, 12, 13, 14, 15, 16, 17, 18, 19, or 20 or more consecutive thymine nucleotides (e.g., a poly-dT portion) within the anchor probe. In some cases, at least some of the thymine nucleotides may be "locked" thymine nucleotides. These may comprise at least 20%, at least 30%, at least 40%, at least 50%, at least 60%, at least 70%, or at least 80% of these thymine nucleotides. In certain embodiments, the locked and non-locked nucleotides may alternate. Such locked thymine nucleotides may be useful, for example, to stabilize the hybridization of the poly-A tails of the mRNA with the anchor probe.

Other methods may be used to anchor nucleic acids, or other molecules in which immobilization is desired. In one set of embodiments, nucleic acids such as DNA or RNA may be immobilized by covalent bonding. For example, in one set of embodiments, an alkylating agent may be used that covalently binds to RNA or DNA and contains a second chemical moiety that can be incorporated into the polyacrylamide as it is polymerized. In yet another set of embodiments, the terminal ribose in an RNA molecule may be oxidized using sodium periodate (or another oxidizing agent) to produce an aldehyde, which may be cross-linked to acrylamide, or other polymer or gel. In other embodiments, chemical agents that are able to modify bases may be used, such as aldehydes, e.g. paraformaldehyde or gluteraldehyde, alkylating agents, or succinimidyl-containing groups; chemical agents that modify the terminal phosphate, such as carboiimides, e.g., EDC (1-ethyl-3-(3-dimethylaminopropyl)carbodiimide); chemical agents that modify internal sugars, such as p-maleimido-phenyl isocyanate; or chemical agents that modify terminal sugars, such as sodium periodate. In some cases, these chemical agents can carry a second chemical moiety that can then be directly cross-linked to the gel or polymer, and/or which can be further modified with a compound that can be directly cross linked to the gel or polymer.

In yet other embodiments, a nucleic acid may be immobilized using anchor probes having substantially complementary portions to the DNA or RNA. There may be 5, 6, 7, 8, 9, 10, 12, 13, 14, 15, 20, 25, 30, 35, 40, 45, 50 or more complementary nucleotides between the anchor probe and the nucleic acid. In still another set of embodiments, the nucleic acids may be physically tangled within the polymer or gel, e.g., due to their length, and, thus, unable to diffuse from their original location within the gel.

Similar anchor probes may be used to immobilize other components to a polymer or gel, in other embodiments. For example, in one set of embodiments, an antibody able to specifically bind to a suitable target (e.g., another protein, a lipid, a carbohydrate, a virus, etc.) may be modified to include an acrydite moiety that can become incorporated within a polymer or gel.

In addition, it should be understood that the embedding of the sample within the matrix and the immobilization of nucleic acids (or other desired targets) may be performed in any suitable order in various embodiments. For instance, immobilization may occur before, during, or after embedding of the sample. In some cases, the target may be chemically modified or reacted to cross-link to the gel or polymer before or during formation of the gel or polymer.

After immobilization of nucleic acids, or other suitable molecules, to the polymer or gel, other components within the sample may be "cleared." Such clearance may include removal of the components, and/or degradation of the components (e.g., to smaller components, components that are not fluorescent, etc.) that are not the desired target. In some cases, at least 50%, at least 60%, at least 70%, at least 80%, or at least 90% of the undesired components within the sample may be cleared. Multiple clearance steps can also be performed in certain embodiments, e.g., to remove various undesired components. As discussed, it is believed that the removal of such components may decrease background during analysis (for example, by decreasing background and/or off-target binding), while desired components (such as nucleic acids) can be immobilized and thus not cleared.

For example, proteins may be cleared from the sample using enzymes, denaturants, chelating agents, chemical agents, and the like, which may break down the proteins into smaller components and/or amino acids. These smaller components may be easier to remove physically, and/or may be sufficiently small or inert such that they do not significantly affect the background. Similarly, lipids may be cleared from the sample using surfactants or the like. In some cases, one or more of these are used, e.g., simultaneously or sequentially. Non-limiting examples of suitable enzymes include proteinases such as proteinase K, proteases or peptidases, or digestive enzymes such as trypsin, pepsin, or chymotrypsin. Non-limiting examples of suitable denaturants include guanidine HCl, acetone, acetic acid, urea, or lithium perchlorate. Non-limiting examples of chemical agents able to denature proteins include solvents such as phenol, chloroform, guanidinium isocyananate, urea, formamide, etc. Non-limiting examples of surfactants include Triton X-100 (polyethylene glycol p-(1,1,3,3-tetramethylbutyl)-phenyl ether), SDS (sodium dodecyl sulfate), Igepal CA-630, or poloxamers. Non-limiting examples of chelating agents include ethylenediaminetetraacetic acid (EDTA), citrate, or polyaspartic acid. In some embodiments, compounds such as these may be applied to the sample to clear proteins, lipids, and/or other components. For instance, a buffer solution (e.g., containing Tris or tris(hydroxymethyl)aminomethane) may be applied to the sample, then removed.

Non-limiting examples of DNA enzymes that may be used to remove DNA include DNase I, dsDNase, a variety of restriction enzymes, etc. Non-limiting examples of techniques to clear RNA include RNA enzymes such as RNase A, RNase T, or RNase H, or chemical agents, e.g., via alkaline hydrolysis (for example, by increasing the pH to greater than 10). Non-limiting examples of systems to remove sugars or extracellular matrix include enzymes such as chitinase, heparinases, or other glycosylases. Non-limiting examples of systems to remove lipids include enzymes such as lipidases, chemical agents such as alcohols (e.g., methanol or ethanol), or detergents such as Triton X-100 or sodium dodecyl sulfate. Many of these are readily available commercially. In this way, the background of the sample may be removed, which may facilitate analysis of the nucleic acid probes or other desired targets, e.g., using fluorescence microscopy, or other techniques as discussed herein. As mentioned, in various embodiments, various targets (e.g., nucleic acids, certain proteins, lipids, viruses, or the like) may be immobilized, while other non-targets may be cleared using suitable agents or enzymes. As a non-limiting example, if a protein (such as an antibody) is immobilized, then RNA enzymes, DNA enzymes, systems to remove lipids, sugars, etc. may be used.

In some cases, the desired target is a nucleic acid. In one set of embodiments, as an illustrative non-limiting example, the sample may be studied by exposing it to one or more types of nucleic acid probes, simultaneously and/or sequentially. For instance, in one set of embodiments, the nucleic acid probes may include smFISH or MERFISH probes, such as those discussed in Int. Pat. Apl. Pub. No. WO 2016/018960 or WO 2016/018963, each incorporated herein by reference in its entirety. However, it should be understood that the following is by way of example only, and in other embodiments, the desired target may be, for example, a protein, a lipid, a virus, or the like.

The nucleic acid probes may comprise nucleic acids (or entities that can hybridize to a nucleic acid, e.g., specifically) such as DNA, RNA, LNA (locked nucleic acids), PNA (peptide nucleic acids), or combinations thereof. In some cases, additional components may also be present within the nucleic acid probes, e.g., as discussed below. Any suitable method may be used to introduce nucleic acid probes into a cell or other sample.

For example, in some embodiments, the cell or other sample is fixed prior to introducing the nucleic acid probes, e.g., to preserve the positions of the nucleic acids within the sample. Techniques for fixing cells and tissues are known to those of ordinary skill in the art. As non-limiting examples, a cell may be fixed using chemicals such as formaldehyde, paraformaldehyde, glutaraldehyde, ethanol, methanol, acetone, acetic acid, or the like. In one embodiment, a cell may be fixed using Hepes-glutamic acid buffer-mediated organic solvent (HOPE).

The nucleic acid probes may be introduced into the cell (or other sample) using any suitable method. In some cases, the cell may be sufficiently permeabilized such that the nucleic acid probes may be introduced into the cell by flowing a fluid containing the nucleic acid probes around the cells. In some cases, the cells may be sufficiently permeabilized as part of a fixation process; in other embodiments, cells may be permeabilized by exposure to certain chemicals such as ethanol, methanol, Triton X-100, or the like. In addition, in some embodiments, techniques such as electroporation or microinjection may be used to introduce nucleic acid probes into a cell or other sample.

Certain aspects of the present invention are generally directed to nucleic acid probes that are introduced into a cell (or other sample). The probes may comprise any of a variety of entities that can hybridize to a nucleic acid, typically by Watson-Crick base pairing, such as DNA, RNA, LNA, PNA, etc., depending on the application. The nucleic acid probe typically contains a target sequence that is able to bind to at least a portion of a target nucleic acid, in some cases specifically. When introduced into a cell or other sample, the nucleic acid probe may be able to bind to a specific target nucleic acid (e.g., an mRNA, or other nucleic acids as discussed herein). In some cases, the nucleic acid probes may be determined using signaling entities (e.g., as discussed below), and/or by using secondary nucleic acid probes able to bind to the nucleic acid probes (i.e., to primary nucleic acid probes). The determination of such nucleic acid probes is discussed in detail below.

In some cases, more than one type of (primary) nucleic acid probe may be applied to a sample, e.g., simultaneously. For example, there may be at least 2, at least 5, at least 10, at least 25, at least 50, at least 75, at least 100, at least 300, at least 1,000, at least 3,000, at least 10,000, at least 30,000, at least 50,000, at least 100,000, at least 250,000, at least 500,000, or at least 1,000,000 distinguishable nucleic acid probes that are applied to a sample, e.g., simultaneously or sequentially.

The target sequence may be positioned anywhere within the nucleic acid probe (or primary nucleic acid probe or encoding nucleic acid probe). The target sequence may contain a region that is substantially complementary to a portion of a target nucleic acid. In some cases, the portions may be at least 50%, at least 60%, at least 70%, at least 75%, at least 80%, at least 85%, at least 90%, at least 92%, at least 94%, at least 95%, at least 96%, at least 97%, at least 98%, at least 99%, or 100% complementary. In some cases, the target sequence may be at least 5, at least 10, at least 15, at least 20, at least 25, at least 30, at least 35, at least 40, at least 50, at least 60, at least 65, at least 75, at least 100, at least 125, at least 150, at least 175, at least 200, at least 250, at least 300, at least 350, at least 400, or at least 450 nucleotides in length. In some cases, the target sequence may be no more than 500, no more than 450, no more than 400, no more than 350, no more than 300, no more than 250, no more than 200, no more than 175, no more than 150, no more than 125, no more than 100, be no more than 75, no more than 60, no more than 65, no more than 60, no more than 55, no more than 50, no more than 45, no more than 40, no more than 35, no more than 30, no more than 20, or no more than 10 nucleotides in length. Combinations of any of these are also possible, e.g., the target sequence may have a length of between 10 and 30 nucleotides, between 20 and 40 nucleotides, between 5 and 50 nucleotides, between 10 and 200 nucleotides, or between 25 and 35 nucleotides, between 10 and 300 nucleotides, etc. Typically, complementarity is determined on the basis of Watson-Crick nucleotide base pairing.

The target sequence of a (primary) nucleic acid probe may be determined with reference to a target nucleic acid suspected of being present within a cell or other sample. For example, a target nucleic acid to a protein may be determined using the protein's sequence, by determining the nucleic acids that are expressed to form the protein. In some cases, only a portion of the nucleic acids encoding the protein are used, e.g., having the lengths as discussed above. In addition, in some cases, more than one target sequence that can be used to identify a particular target may be used. For instance, multiple probes can be used, sequentially and/or simultaneously, that can bind to or hybridize to different regions of the same target. Hybridization typically refers to an annealing process by which complementary single-stranded nucleic acids associate through Watson-Crick nucleotide base pairing (e.g., hydrogen bonding, guanine-cytosine and adenine-thymine) to form double-stranded nucleic acid.

In some embodiments, a nucleic acid probe, such as a primary nucleic acid probe, may also comprise one or more "read" sequences. However, it should be understood that read sequences are not necessary in all cases. In some embodiments, the nucleic acid probe may comprise 1, 2, 3, 4, 5, 6, 7, 8, 9, 10, 11, 12, 13, 14, 15, 16 or more, 20 or more, 32 or more, 40 or more, 50 or more, 64 or more, 75 or more, 100 or more, 128 or more read sequences. The read sequences may be positioned anywhere within the nucleic acid probe. If more than one read sequence is present, the read sequences may be positioned next to each other, and/or interspersed with other sequences.

The read sequences, if present, may be of any length. If more than one read sequence is used, the read sequences may independently have the same or different lengths. For instance, the read sequence may be at least 5, at least 10, at least 15, at least 20, at least 25, at least 30, at least 35, at least 40, at least 50, at least 60, at least 65, at least 75, at least 100, at least 125, at least 150, at least 175, at least 200, at least 250, at least 300, at least 350, at least 400, or at least 450 nucleotides in length. In some cases, the read sequence may be no more than 500, no more than 450, no more than 400, no more than 350, no more than 300, no more than 250, no more than 200, no more than 175, no more than 150, no more than 125, no more than 100, be no more than 75, no more than 60, no more than 65, no more than 60, no more than 55, no more than 50, no more than 45, no more than 40, no more than 35, no more than 30, no more than 20, or no more than 10 nucleotides in length. Combinations of any of these are also possible, e.g., the read sequence may have a length of between 10 and 30 nucleotides, between 20 and 40 nucleotides, between 5 and 50 nucleotides, between 10 and 200 nucleotides, or between 25 and 35 nucleotides, between 10 and 300 nucleotides, etc.

The read sequence may be arbitrary or random in some embodiments. In certain cases, the read sequences are chosen so as to reduce or minimize homology with other components of the cell or other sample, e.g., such that the read sequences do not themselves bind to or hybridize with other nucleic acids suspected of being within the cell or other sample. In some cases, the homology may be less than 10%, less than 8%, less than 7%, less than 6%, less than 5%, less than 4%, less than 3%, less than 2%, or less than 1%. In some cases, there may be a homology of less than 20 basepairs, less than 18 basepairs, less than 15 basepairs, less than 14 basepairs, less than 13 basepairs, less than 12 basepairs, less than 11 basepairs, or less than 10 basepairs. In some cases, the basepairs are sequential.

In one set of embodiments, a population of nucleic acid probes may contain a certain number of read sequences, which may be less than the number of targets of the nucleic acid probes in some cases. Those of ordinary skill in the art will be aware that if there is one signaling entity and $n$ read sequences, then in general $2^n-1$ different nucleic acid targets may be uniquely identified. However, not all possible combinations need be used. For instance, a population of nucleic acid probes may target 12 different nucleic acid sequences, yet contain no more than 8 read sequences. As another example, a population of nucleic acids may target 140 different nucleic acid species, yet contain no more than 16 read sequences. Different nucleic acid sequence targets may be separately identified by using different combinations of read sequences within each probe. For instance, each probe may contain 1, 2, 3, 4, 5, 6, 7, 8, 9, 10, 11, 12, 13, 14, 15, etc. or more read sequences. In some cases, a population of nucleic acid probes may each contain the same number of read sequences, although in other cases, there may be different numbers of read sequences present on the various probes.

As a non-limiting example, a first nucleic acid probe may contain a first target sequence, a first read sequence, and a second read sequence, while a second, different nucleic acid probe may contain a second target sequence, the same first read sequence, but a third read sequence instead of the second read sequence. Such probes may thereby be distinguished by determining the various read sequences present or associated with a given probe or location, as discussed herein.

In addition, the nucleic acid probes (and their corresponding, complimentary sites on the encoding probes), in certain embodiments, may be made using only 2 or only 3 of the 4 bases, such as leaving out all the "G"s or leaving out all of the "C"s within the probe. Sequences lacking either "G"s or "C"s may form very little secondary structure in certain embodiments, and can contribute to more uniform, faster hybridization.

In some embodiments, the nucleic acid probe may contain a signaling entity. It should be understood that signaling entities are not required in all cases, however; for instance, the nucleic acid probe may be determined using secondary nucleic acid probes in some embodiments, as is discussed in additional detail below. Examples of signaling entities that can be used are also discussed in more detail below.

Other components may also be present within a nucleic acid probe as well. For example, in one set of embodiments, one or more primer sequences may be present, e.g., to allow for enzymatic amplification of probes. Those of ordinary skill in the art will be aware of primer sequences suitable for applications such as amplification (e.g., using PCR or other suitable techniques). Many such primer sequences are available commercially. Other examples of sequences that may be present within a primary nucleic acid probe include, but are not limited to promoter sequences, operons, identification sequences, nonsense sequences, or the like.

Typically, a primer is a single-stranded or partially double-stranded nucleic acid (e.g., DNA) that serves as a starting point for nucleic acid synthesis, allowing polymerase enzymes such as nucleic acid polymerase to extend the primer and replicate the complementary strand. A primer is (e.g., is designed to be) complementary to and to hybridize to a target nucleic acid. In some embodiments, a primer is a synthetic primer. In some embodiments, a primer is a non-naturally-occurring primer. A primer typically has a length of 10 to 50 nucleotides. For example, a primer may have a length of 10 to 40, 10 to 30, 10 to 20, 25 to 50, 15 to 40, 15 to 30, 20 to 50, 20 to 40, or 20 to 30 nucleotides. In some embodiments, a primer has a length of 18 to 24 nucleotides.

In addition, the components of the nucleic acid probe may be arranged in any suitable order. For instance, in one embodiment, the components may be arranged in a nucleic acid probe as: primer-read sequences-targeting sequence-read sequences-reverse primer. The "read sequences" in this structure may each contain any number (including 0) of read sequences, so long as at least one read sequence is present in the probe. Non-limiting example structures include primer-targeting sequence-read sequences-reverse primer, primer-read sequences-targeting sequence-reverse primer, targeting sequence-primer-targeting sequence-read sequences-reverse primer, targeting sequence-primer-read sequences-targeting sequence-reverse primer, primer-target sequence-read sequences-targeting sequence-reverse primer, targeting sequence-primer-read sequence-reverse primer, targeting sequence-read sequence-primer, read sequence-targeting sequence-primer, read sequence-primer-targeting sequence-reverse primer, etc. In addition, the reverse primer is optional in some embodiments, including in all of the above-described examples.

After introduction of the nucleic acid probes into a cell or other sample, the nucleic acid probes may be directly determined by determining signaling entities (if present), and/or the nucleic acid probes may be determined by using one or more secondary nucleic acid probes, in accordance with certain aspects of the invention. As mentioned, in some cases, the determination may be spatial, e.g., in two or three dimensions. In addition, in some cases, the determination may be quantitative, e.g., the amount or concentration of a primary nucleic acid probe (and of a target nucleic acid) may be determined. Additionally, the secondary probes may comprise any of a variety of entities able to hybridize a nucleic acid, e.g., DNA, RNA, LNA, and/or PNA, etc., depending on the application. Signaling entities are discussed in more detail below.

A secondary nucleic acid probe may contain a recognition sequence able to bind to or hybridize with a read sequence of a primary nucleic acid probe. In some cases, the binding is specific, or the binding may be such that a recognition sequence preferentially binds to or hybridizes with only one of the read sequences that are present. The secondary nucleic acid probe may also contain one or more signaling entities. If more than one secondary nucleic acid probe is used, the signaling entities may be the same or different.

The recognition sequences may be of any length, and multiple recognition sequences may be of the same or different lengths. If more than one recognition sequence is used, the recognition sequences may independently have the same or different lengths. For instance, the recognition sequence may be at least 5, at least 10, at least 15, at least 20, at least 25, at least 30, at least 35, at least 40, or at least 50 nucleotides in length. In some cases, the recognition sequence may be no more than 75, no more than 60, no more than 65, no more than 60, no more than 55, no more than 50, no more than 45, no more than 40, no more than 35, no more than 30, no more than 20, or no more than 10 nucleotides in length. Combinations of any of these are also possible, e.g., the recognition sequence may have a length of between 10 and 30, between 20 and 40, or between 25 and 35 nucleotides, etc. In one embodiment, the recognition sequence is of the same length as the read sequence. In addition, in some cases, the recognition sequence may be at least 50%, at least 60%, at least 70%, at least 75%, at least 80%, at least 85%, at least 90%, at least 92%, at least 94%, at least 95%, at least 96%, at least 97%, at least 98%, at least 99%, or at least 100% complementary to a read sequence of the primary nucleic acid probe.

As mentioned, in some cases, the secondary nucleic acid probe may comprise one or more signaling entities. Examples of signaling entities are discussed in more detail below.

As discussed, in certain aspects of the invention, nucleic acid probes are used that contain various "read sequences." For example, a population of primary nucleic acid probes may contain certain "read sequences" which can bind certain of the secondary nucleic acid probes, and the locations of the primary nucleic acid probes are determined within the sample using secondary nucleic acid probes, e.g., which comprise a signaling entity. As mentioned, in some cases, a population of read sequences may be combined in various combinations to produce different nucleic acid probes, e.g., such that a relatively small number of read sequences may be used to produce a relatively large number of different nucleic acid probes.

Thus, in some cases, a population of primary nucleic acid probes (or other nucleic acid probes) may each contain a certain number of read sequences, some of which are shared between different primary nucleic acid probes such that the total population of primary nucleic acid probes may contain a certain number of read sequences. A population of nucleic acid probes may have any suitable number of read sequences. For example, a population of primary nucleic acid probes may have 1, 2, 3, 4, 5, 6, 7, 8, 9, 10, 11, 12, 13, 14, 15, 16, 17, 18, 19, 20 etc. read sequences. More than 20 are also possible in some embodiments. In addition, in some cases, a population of nucleic acid probes may, in total, have 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 20 or more, 24 or more, 32 or more, 40 or more, 50 or more, 60 or more, 64 or more, 100 or more, 128 or more, etc. of possible read sequences present, although some or all of the probes may each contain more than one read sequence, as discussed herein. In addition, in some embodiments, the population of nucleic acid probes may have no more than 100, no more than 80, no more than 64, no more than 60, no more than 50, no more than 40, no more than 32, no more than 24, no more than 20, no more than 16, no more than 15, no more than 14, no more than 13, no more than 12, no more than 11, no more than 10, no more than 9, no more than 8, no more than 7, no more than 6, no more than 5, no more than 4, no more than 3, or no more than two read sequences present. Combinations of any of these are also possible, e.g., a population of nucleic acid probes may comprise between 10 and 15 read sequences in total.

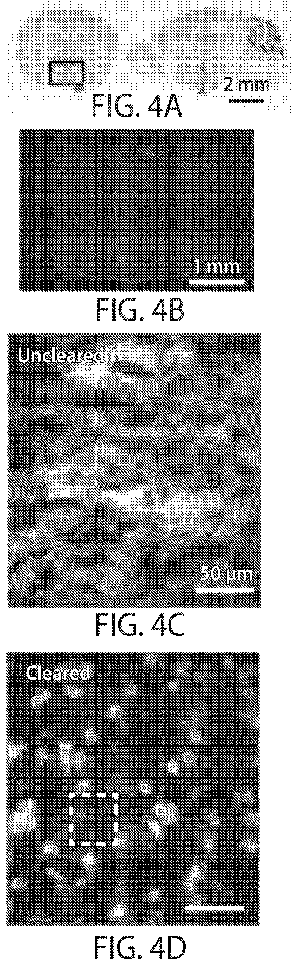

As a non-limiting example of an approach to combinatorially producing a relatively large number of nucleic acid probes from a relatively small number of read sequences, in a population of 6 different types of nucleic acid probes, each comprising one or more read sequences, the total number of read sequences within the population may be no greater than 4. It should be understood that although 4 read sequences are used in this example for ease of explanation, in other embodiments, larger numbers of nucleic acid probes may be realized, for example, using 5, 8, 10, 16, 32, etc. or more read sequences, or any other suitable number of read sequences described herein, depending on the application. If each of the primary nucleic acid probes contains two different read sequences, then by using 4 such read sequences (A, B, C, and D), up to 6 probes may be separately identified. It should be noted that in this example, the ordering of read sequences on a nucleic acid probe is not essential, i.e., "AB" and "BA" may be treated as being synonymous (although in other embodiments, the ordering of read sequences may be essential and "AB" and "BA" may not necessarily be synonymous). Similarly, if 5 read sequences are used (A, B, C, D, and E) in the population of primary nucleic acid probes, up to 10 probes may be separately identified, as is shown in Fig. 4B. For example, one of ordinary skill in the art would understand that, for $k$ read sequences in a population with $n$ read sequences on each probe, up to $\binom{n}{k}$ different probes may be produced, assuming that the ordering of read sequences is not essential; because not all of the probes need to have the same number of read sequences and not all combinations of read sequences need to be used in every embodiment, either more or less than this number of different probes may also be used in certain embodiments. In addition, it should also be understood that the number of read sequences on each probe need not be identical in some embodiments. For instance example, some probes may contain 2 read sequences while other probes may contain 3 read sequences.

In some aspects, the read sequences and/or the pattern of binding of nucleic acid probes within a sample may be used to define an error-detecting and/or an error-correcting code, for example, to reduce or prevent misidentification or errors of the nucleic acids. Thus, for example, if binding is indicated (e.g., as determined using a signaling entity), then the location may be identified with a "1"; conversely, if no binding is indicated, then the location may be identified with a "0" (or vice versa, in some cases). Multiple rounds of binding determinations, e.g., using different nucleic acid probes, can then be used to create a "codeword," e.g., for that spatial location. In some embodiments, the codeword may be subjected to error detection and/or correction. For instance, the codewords may be organized such that, if no match is found for a given set of read sequences or binding pattern of nucleic acid probes, then the match may be identified as an error, and optionally, error correction may be applied sequences to determine the correct target for the nucleic acid probes. In some cases, the codewords may have fewer "letters" or positions that the total number of nucleic acids encoded by the codewords, e.g. where each codeword encodes a different nucleic acid.

Such error-detecting and/or the error-correction code may take a variety of forms. A variety of such codes have previously been developed in other contexts such as the telecommunications industry, such as Golay codes or Hamming codes. In one set of embodiments, the read sequences or binding patterns of the nucleic acid probes are assigned such that not every possible combination is assigned.

For example, if 4 read sequences are possible and a primary nucleic acid probe contains 2 read sequences, then up to 6 primary nucleic acid probes could be identified; but the number of primary nucleic acid probes used may be less than 6. Similarly, for $k$ read sequences in a population with $n$ read sequences on each primary nucleic acid probe, $\binom{n}{k}$ different probes may be produced, but the number of primary nucleic acid probes that are used may be any number more or less than $\binom{n}{k}$. In addition, these may be randomly assigned, or assigned in specific ways to increase the ability to detect and/or correct errors.

As another example, if multiple rounds of nucleic acid probes are used, the number of rounds may be arbitrarily chosen. If in each round, each target can give two possible outcomes, such as being detected or not being detected,

up to $2^n$ different targets may be possible for $n$ rounds of probes, but the number of nucleic acid targets that are actually used may be any number less than $2^n$. For example, if in each round, each target can give more than two possible outcomes, such as being detected in different color channels, more than $2^n$ (e.g. $3^n$, $4^n$ ...) different targets may be possible for $n$ rounds of probes. In some cases, the number of nucleic acid targets that are actually used may be any number less than this number. In addition, these may be randomly assigned, or assigned in specific ways to increase the ability to detect and/or correct errors.

For example, in one set of embodiments, the codewords or nucleic acid probes may be assigned within a code space such that the assignments are separated by a Hamming distance, which measures the number of incorrect "reads" in a given pattern that cause the nucleic acid probe to be misinterpreted as a different valid nucleic acid probe. In certain cases, the Hamming distance may be at least 2, at least 3, at least 4, at least 5, at least 6, or the like. In addition, in one set of embodiments, the assignments may be formed as a Hamming code, for instance, a Hamming(7, 4) code, a Hamming(15, 11) code, a Hamming(31, 26) code, a Hamming(63, 57) code, a Hamming(127, 120) code, etc. In another set of embodiments, the assignments may form a SECDED code, e.g., a SECDED(8,4) code, a SECDED(16,4) code, a SCEDED(16, 11) code, a SCEDED(22, 16) code, a SCEDED(39, 32) code, a SCEDED(72, 64) code, etc. In yet another set of embodiments, the assignments may form an extended binary Golay code, a perfect binary Golay code, or a ternary Golay code. In another set of embodiments, the assignments may represent a subset of the possible values taken from any of the codes described above.

For example, a code with the same error correcting properties of the SECDED code may be formed by using only binary words that contain a fixed number of '1' bits, such as 4, to encode the targets. In another set of embodiments, the assignments may represent a subset of the possible values taken from codes described above for the purpose of addressing asymmetric readout errors. For example, in some cases, a code in which the number of '1' bits may be fixed for all used binary words may eliminate the biased measurement of words with different numbers of '1's when the rate at which '0' bits are measured as '1' s or '1' bits are measured as '0's are different.

Accordingly, in some embodiments, once the codeword is determined (e.g., as discussed herein), the codeword may be compared to the known nucleic acid codewords. If a match is found, then the nucleic acid target can be identified or determined. If no match is found, then an error in the reading of the codeword may be identified. In some cases, error correction can also be applied to determine the correct codeword, and thus resulting in the correct identity of the nucleic acid target. In some cases, the codewords may be selected such that, assuming that there is only one error present, only one possible correct codeword is available, and thus, only one correct identity of the nucleic acid target is possible. In some cases, this may also be generalized to larger codeword spacings or Hamming distances; for instance, the codewords may be selected such that if two, three, or four errors are present (or more in some cases), only one possible correct codeword is available, and thus, only one correct identity of the nucleic acid targets is possible.

The error-correcting code may be a binary error-correcting code, or it may be based on other numbering systems, e.g., ternary or quaternary error-correcting codes. For instance, in one set of embodiments, more than one type of signaling entity may be used and assigned to different numbers within the error-correcting code. Thus, as a non-limiting example, a first signaling entity (or more than one signaling entity, in some cases) may be assigned as "1" and a second signaling entity (or more than one signaling entity, in some cases) may be assigned as "2" (with "0" indicating no signaling entity present), and the codewords distributed to define a ternary error-correcting code. Similarly, a third signaling entity may additionally be assigned as "3" to make a quaternary error-correcting code, etc.

As discussed above, in certain aspects, signaling entities are determined, e.g., to determine nucleic acid probes and/or to create codewords. In some cases, signaling entities within a sample may be determined, e.g., spatially, using a variety of techniques. In some embodiments, the signaling entities may be fluorescent, and techniques for determining fluorescence within a sample, such as fluorescence microscopy or confocal microscopy, may be used to spatially identify the positions of signaling entities within a cell. In some cases, the positions of entities within the sample may be determined in two or even three dimensions. In addition, in some embodiments, more than one signaling entity may be determined at a time (e.g., signaling entities with different colors or emissions), and/or sequentially.

In addition, in some embodiments, a confidence level for the identified nucleic acid target may be determined. For example, the confidence level may be determined using a ratio of the number of exact matches to the number of matches having one or more one-bit errors. In some cases, only matches having a confidence ratio greater than a certain value may be used. For instance, in certain embodiments, matches may be accepted only if the confidence ratio for the match is greater than about 0.01, greater than about 0.03, greater than about 0.05, greater than about 0.1, greater than about 0.3, greater than about 0.5, greater than about 1, greater than about 3, greater than about 5, greater than about 10, greater than about 30, greater than about 50, greater than about 100, greater than about 300, greater than about 500, greater than about 1000, or any other suitable value. In addition, in some embodiments, matches may be accepted only if the confidence ratio for the identified nucleic acid target is greater than an internal standard or false positive control by about 0.01, about 0.03, about 0.05, about 0.1, about 0.3, about 0.5, about 1, about 3, about 5, about 10, about 30, about 50, about 100, about 300, about 500, about 1000, or any other suitable value

In some embodiments, the spatial positions of the entities (and thus, nucleic acid probes that the entities may be associated with) may be determined at relatively high resolutions. For instance, the positions may be determined at spatial resolutions of better than about 100 micrometers, better than about 30 micrometers, better than about 10 micrometers, better than about 3 micrometers, better than about 1 micrometer, better than about 800 nm, better than about 600 nm, better than about 500 nm, better than about 400 nm, better than about 300 nm, better than about 200 nm, better than about 100 nm, better than about 90 nm, better than about 80 nm, better than about 70 nm, better than about 60 nm, better than about 50 nm, better than about 40 nm, better than about 30 nm, better than about 20 nm, or better than about 10 nm, etc.

There are a variety of techniques able to determine or image the spatial positions of entities or targets optically, e.g., using fluorescence microscopy, using radioactivity, via conjugation with suitable chromophores, or the like. For example, various conventional microscopy techniques that may be used in various embodiments of the invention include, but are not limited to, epi-fluorescence microscopy, total-internal-reflectance microscopy, highly-inclined thin-illumination (HILO) microscopy, light-sheet microscopy, scanning confocal microscopy, scanning line confocal microscopy, spinning disk confocal microscopy, or other comparable conventional microscopy techniques.

In some embodiments, *in situ* hybridization (ISH) techniques for labeling nucleic acids such as DNA or RNA may be used, e.g., where nucleic acid probes may be hybridized to nucleic acids in samples. These may be performed, e.g., at cellular-scale or single-molecule-scale resolutions. In some cases, the ISH probes can be composed of RNA, DNA, PNA, LNA, other synthetic nucleotides, or the like, and/or a combination of any of these. The presence of a hybridized probe can be measured, for example, with radioactivity using radioactively labeled nucleic acid probes, immunohistochemistry using, for example, biotin labeled nucleic acid probes, enzymatic chromophore or fluorophore generation using, for example, probes that can bind enzymes such as horseradish peroxidase and approaches such as tyramide signal amplification, fluorescence imaging using nucleic acid probes directly labeled with fluorophores, or hybridization of secondary nucleic acid probes to these primary probes, with the secondary probes detected via any of the above methods.

In some cases, the spatial positions may be determined at super resolutions, or at resolutions better than the wavelength of light or the diffraction limit (although in other embodiments, super resolutions are not required). Non-limiting examples include STORM (stochastic optical reconstruction microscopy), STED (stimulated emission depletion microscopy), NSOM (Near-field Scanning Optical Microscopy), 4Pi microscopy, SIM (Structured Illumination Microscopy), SMI (Spatially Modulated Illumination) microscopy, RESOLFT (Reversible Saturable Optically Linear Fluorescence Transition Microscopy), GSD (Ground State Depletion Microscopy), SSIM (Saturated Structured-Illumination Microscopy), SPDM (Spectral Precision Distance Microscopy), Photo-Activated Localization Microscopy (PALM), Fluorescence Photo-toactivation Localization Microscopy (FPALM), LIMON (3D Light Microscopical Nanosizing Microscopy), Super-resolution optical fluctuation imaging (SOFI), or the like. See, e.g., U.S. Pat. No. 7,838,302, issued November 23, 2010, entitled "Sub-Diffraction Limit Image Resolution and Other Imaging Techniques," by Zhuang, et al.; U.S. Pat. No. 8,564,792, issued October 22, 2013, entitled "Sub-diffraction Limit Image Resolution in Three Dimensions," by Zhuang, et al.; or Int. Pat. Apl. Pub. No. WO 2013/090360, published June 20, 2013, entitled "High Resolution Dual-Objective Microscopy," by Zhuang, et al., each incorporated herein by reference in their entireties.

In one embodiment, the sample may be illuminated by single Gaussian mode laser lines. In some embodiments, the illumination profiled may be flattened by passing these laser lines through a multimode fiber that is vibrated via piezo-electric or other mechanical means. In some embodiments, the illumination profile may be flattened by passing single-mode, Gaussian beams through a variety of refractive beam shapers, such as the piShaper or a series of stacked Powell lenses. In yet another set of embodiments, the Gaussian beams may be passed through a variety of different diffusing elements, such as ground glass or engineered diffusers, which may be spun in some cases at high speeds to remove residual laser speckle. In yet another embodiment, laser illumination may be passed through a series of lenslet arrays to produce overlapping images of the illumination that approximate a flat illumination field.

In some embodiments, the centroids of the spatial positions of the entities may be determined. For example, a centroid of a signaling entity may be determined within an image or series of images using image analysis algorithms known to those of ordinary skill in the art. In some cases, the algorithms may be selected to determine non-overlapping single emitters and/or partially overlapping single emitters in a sample. Non-limiting examples of suitable techniques include a maximum likelihood algorithm, a least squares algorithm, a Bayesian algorithm, a compressed sensing algorithm, or the like. Combinations of these techniques may also be used in some cases.

In addition, the signaling entity may be inactivated in some cases. For example, in some embodiments, a first secondary nucleic acid probe containing a signaling entity may be applied to a sample that can recognize a first read sequence, then the first secondary nucleic acid probe can be inactivated before a second secondary nucleic acid probe is applied to the sample. If multiple signaling entities are used, the same or different techniques may be used to inactivate the signaling entities, and some or all of the multiple signaling entities may be inactivated, e.g., sequentially or simultaneously.

Inactivation may be caused by removal of the signaling entity (e.g., from the sample, or from the nucleic acid probe, etc.), and/or by chemically altering the signaling entity in some fashion, e.g., by photobleaching the signaling entity, bleaching or chemically altering the structure of the signaling entity, e.g., by reduction, etc.). For instance, in one set of embodiments, a fluorescent signaling entity may be inactivated by chemical or optical techniques such as oxidation, photobleaching, chemically bleaching, stringent washing or enzymatic digestion or reaction by exposure to an enzyme, dissociating the signaling entity from other components (e.g., a probe), chemical reaction of the signaling entity (e.g., to a reactant able to alter the structure of the signaling entity) or the like. For instance, bleaching may occur by exposure to oxygen, reducing agents, or the signaling entity could be chemically cleaved from the nucleic acid probe and washed away via fluid flow.

In some embodiments, various nucleic acid probes (including primary and/or secondary nucleic acid probes) may include one or more signaling entities. If more than one nucleic acid probe is used, the signaling entities may each by the same or different. In certain embodiments, a signaling entity is any entity able to emit light. For instance, in one embodiment, the signaling entity is fluorescent. In other embodiments, the signaling entity may be phosphorescent, radioactive, absorptive, etc. In some cases, the signaling entity is any entity that can be determined within a sample at relatively high resolutions, e.g., at resolutions better than the wavelength of visible light or the diffraction limit. The signaling entity may be, for example, a dye, a small molecule, a peptide or protein, or the like. The signaling entity may be a single molecule in some cases. If multiple secondary nucleic acid probes are used, the nucleic acid probes may comprise the same or different signaling entities.

Non-limiting examples of signaling entities include fluorescent entities (fluorophores) or phosphorescent entities, for example, cyanine dyes (e.g., Cy2, Cy3, Cy3B, Cy5, Cy5.5, Cy7, etc.), Alexa Fluor dyes, Atto dyes, photoswtichable dyes, photoactivatable dyes, fluorescent dyes, metal nanoparticles, semiconductor nanoparticles or "quantum dots", fluorescent proteins such as GFP (Green Fluorescent Protein), or photoactivabale fluorescent proteins, such as PAGFP, PSCFP, PSCFP2, Dendra, Dendra2, EosFP, tdEos, mEos2, mEos3, PAmCherry, PAtagRFP, mMaple, mMaple2, and mMaple3. Other suitable signaling entities are known to those of ordinary skill in the art. See, e.g., U.S. Pat. No. 7,838,302 or U.S. Pat. Apl. Ser. No. 61/979,436, each incorporated herein by reference in its entirety. In some cases, spectrally distinct fluorescent dyes may be used.

In one set of embodiments, the signaling entity may be attached to an oligonucleotide sequence via a bond that can be cleaved to release the signaling entity. In one set of embodiments, a fluorophore may be conjugated to an oligonucleotide via a cleavable bond, such as a photocleavable bond. Non-limiting examples of photocleavable bonds include, but are not limited to, 1-(2-nitrophenyl)ethyl, 2-nitrobenzyl, biotin phosphoramidite, acrylic phosphoramidite, diethylaminocoumarin, 1-(4,5-dimethoxy-2-nitrophenyl)ethyl, cyclododecyl (dimethoxy-2-nitrophenyl)ethyl, 4-aminomethyl-3-nitrobenzyl, (4-nitro-3-(1-chlorocarbonyloxyethyl)phenyl)methyl-S-acetylthioic acid ester, (4-nitro-3-(1-thlorocarbonyloxyethyl)phenyl)methyl-3-(2-pyridyldithiopropionic acid) ester, 3-(4,4'-dimethoxytrityl)-1-(2-nitrophenyl)-propane-1,3-diol-[2-    cyanoethyl-(N,N-diisopropyl)]-phosphoramidite,    1-[2-nitro-5-(6-trifluoroacetylcaproamidomethyl)phenyl] -ethyl-[2-cyanoethyl-(N,N-diisopropyl)]-phosphoramidite,    1-[2-nitro-5-(6-(4,4'-dimethoxytrityloxy)butyramidomethyl)phenyl]-ethyl-[2-cyanoethyl-(N,N-diisopropyl)]-phosphoramidite, 1-[2-nitro-5-(6-(N-(4,4'-dimethoxytrityl))-biotinamidocaproamido-methyl)phenyl]-ethyl-[2-cyanoethyl-(N,N-diisopropyl)]-phosphoramidite, or similar linkers. In another set of embodiments, the fluorophore may be conjugated to an oligonucleotide via a disulfide bond. The disulfide bond may be cleaved by a variety of reducing agents such as, but not limited to, dithiothreitol, dithioerythritol, beta-mercaptoethanol, sodium borohydride, thioredoxin, glutaredoxin, trypsinogen, hydrazine, diisobutylaluminum hydride, oxalic acid, formic acid, ascorbic acid, phosphorous acid, tin chloride, glutathione, thioglycolate, 2,3-dimercaptopropanol, 2-mercaptoethylamine, 2-aminoethanol, tris(2-carboxyethyl)phosphine, bis(2-mercaptoethyl) sulfone, N,N'-dimethyl-N,N',-bis(mercaptoacetyl)hydrazine, 3-mercaptoproptionate, dimethylformamide, thiopropyl-agarose, tri-*n*-butylphosphine, cysteine, iron sulfate, sodium sulfite, phosphite, hypophosphite, phosphorothioate, or the like, and/or combinations of any of these. In another embodiment, the fluorophore may be conjugated to an oligonucleotide via one or more phosphorothioate modified nucleotides in which the sulfur modification replaces the bridging and/or non-bridging oxygen. The fluorophore may be cleaved from the oligonucleotide, in certain embodiments, via addition of compounds such as but not limited to iodoethanol, iodine mixed in ethanol, silver nitrate, or mercury chloride. In yet another set of embodiments, the signaling entity may be chemically inactivated through reduction or oxidation. For example, in one embodiment, a chromophore such as Cy5 or Cy7 may be reduced using sodium borohydride to a stable, non-fluorescence state. In still another set of embodiments, a fluorophore may be conjugated to an oligonucleotide via an azo bond, and the azo bond may be cleaved with 2-[(2-N-arylamino)phenylazo]pyridine. In yet another set of embodiments, a fluorophore may be conjugated to an oligonucleotide via a suitable nucleic acid segment that can be cleaved upon suitable exposure to DNAse, e.g., an exodeoxyribonuclease or an endodeoxyribonuclease. Examples include, but are not limited to, deoxyribonuclease I or deoxyribonuclease II. In one set of embodiments, the cleavage may occur via a restriction endonuclease. Non-limiting examples of potentially suitable restriction endonucleases include BamHI, BsrI, NotI, XmaI, PspAI, DpnI, MboI, MnlI, Eco57I, Ksp632I, DraIII, AhaII, SmaI, MluI, HpaI, ApaI, BclI, BstEII, TaqI, EcoRI, SacI, HindII, HaeII, DraII, Tsp509I, Sau3AI, PacI, etc. Over 3000 restriction enzymes have been studied in detail, and more than 600 of these are available commercially. In yet another set of embodiments, a fluorophore may be conjugated to biotin, and the oligonucleotide conjugated to avidin or streptavidin. An interaction between biotin and avidin or streptavidin allows the fluorophore to be conjugated to the oligonucleotide, while sufficient exposure to an excess of addition, free biotin could "outcompete" the linkage and thereby cause cleavage to occur. In addition, in another set of embodiments, the probes may be removed using corresponding "toe-hold-probes," which comprise the same sequence as the probe, as well as an extra number of bases of homology to the encoding probes (e.g., 1-20 extra bases, for example, 5 extra bases). These probes may remove the labeled readout probe through a strand-displacement interaction.

As used herein, the term "light" generally refers to electromagnetic radiation, having any suitable wavelength (or equivalently, frequency). For instance, in some embodiments, the light may include wavelengths in the optical or visual range (for example, having a wavelength of between about 400 nm and about 700 nm, i.e., "visible light"), infrared wavelengths (for example, having a wavelength of between about 300 micrometers and 700 nm), ultraviolet wavelengths (for example, having a wavelength of between about 400 nm and about 10 nm), or the like. In certain cases, as discussed in detail below, more than one entity may be used, i.e., entities that are chemically different or distinct, for example, structurally. However, in other cases, the entities may be chemically identical or at least substantially chemically identical.

Another aspect of the invention is directed to a computer-implemented method. For instance, a computer and/or an automated system may be provided that is able to automatically and/or repetitively perform any of the methods described herein. As used herein, "automated" devices refer to devices that are able to operate without human direction, i.e., an automated device can perform a function during a period of time after any human has finished taking any action to promote the function, e.g. by entering instructions into a computer to start the process. Typically, automated equipment can perform repetitive functions after this point in time. The processing steps may also be recorded onto a machine-readable medium in some cases.

For example, in some cases, a computer may be used to control imaging of the sample, e.g., using fluorescence microscopy, STORM or other super-resolution techniques such as those described herein. In some cases, the computer may also control operations such as drift correction, physical registration, hybridization and cluster alignment in image analysis, cluster decoding (e.g., fluorescent cluster decoding), error detection or correction (e.g., as discussed herein), noise reduction, identification of foreground features from background features (such as noise or debris in images), or the like. As an example, the computer may be used to control activation and/or excitation of signaling entities within the sample, and/or the acquisition of images of the signaling entities. In one set of embodiments, a sample may be excited using light having various wavelengths and/or intensities, and the sequence of the wavelengths of light used to excite the sample may be correlated, using a computer, to the images acquired of the sample containing the signaling entities. For instance, the computer may apply light having various wavelengths and/or intensities to a sample to yield different average numbers of signaling entities in each region of interest (e.g., one activated entity per location, two activated entities per location, etc.). In some cases, this information may be used to construct an image and/or determine the locations of the signaling entities, in some cases at high resolutions, as noted above.

The following are incorporated herein by reference: International Patent Application No. PCT/US2015/042556, filed July 29, 2015, entitled "Systems and Methods for Determining Nucleic Acids," by Zhuang, et al., published as WO 2016/018960 on February 4, 2016; International Patent Application No. PCT/US2015/042559, filed July 29, 2015, entitled "Probe Library Construction," by Zhuang, et al., published as WO 2016/018963 on February 4, 2016; and U.S. Provisional Patent Application Serial No. 62/419,033, filed November 8, 2016, entitled "Matrix Imprinting and Clearing," by Zhuang, et al.

The following examples are intended to illustrate certain embodiments of the present invention, but do not exemplify the full scope of the invention.


**EXAMPLE 1**

This example illustrates a sample clearing approach for FISH measurements. These examples identify off-target binding of FISH probes to cellular components other than RNA, such as proteins, as the major source of background. To remove this source of background, samples were embedded in polyacrylamide (PA), anchored RNAs to this PA matrix, and cellular proteins and lipids, which are also sources of autofluorescence, were cleared. Additional details are provided in Example 5. To demonstrate the efficacy of this approach, this example measured the copy number of 130 RNAs in cleared samples using multiplexed error-robust fluorescence in situ hybridization (MERFISH). A reduction was observed both in the background due to off-target probe binding, and in the cellular autofluorescence without detectable loss in RNA. This led to an improved detection efficiency and detection limit of MERFISH, and an increased measurement throughput via extension of MERFISH into four color channels. These examples further demonstrate MERFISH measurements of complex tissue samples from the mouse brain using this matrix imprinting and clearing approach. It is expected that this will improve the performance of a wide range of *in situ*-hybridization-based techniques in both cell culture and tissues.

Single-molecule fluorescence *in situ* hybridization (smFISH) is a powerful technique that allows the direct imaging of individual RNAs within single cells. In this approach, individual copies of a specific RNA species are labeled via the hybridization of fluorescently labeled oligonucleotide probes, producing bright fluorescent spots for single RNA molecules, which reveal both the abundance and the spatial distribution of these RNAs inside cells. The ability to image gene expression at the single-cell level in both cell culture and tissue has led to exciting advances in understanding the natural noise in gene expression and its role in cellular response, the intracellular spatial organization of RNAs and its role in post-transcriptional regulation, and the natural spatial variation in gene expression within complex tissues and its role in the molecular definition of cell type and tissue function.

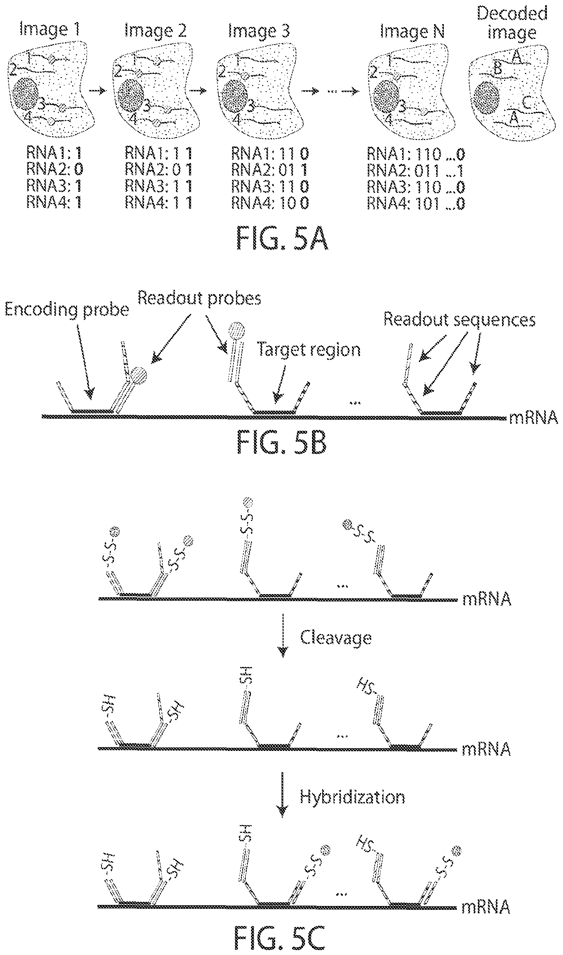

In order to extend the benefits of this technique to systems-level questions and high-throughput gene expression profiling, approaches to increase the multiplexing of smFISH-i.e. the number of different RNA species that can be simultaneously quantified within the same cell-have been developed. Most of these approaches take advantage of color multiplexing which has allowed tens of RNA species to be imaged simultaneously. Multiplexed error robust fluorescence *in situ* hybridization (MERFISH) is a massively multiplexed form of smFISH that allows RNA imaging and profiling at the transcriptomic scale. See, for example, Int. Pat. Apl. Pub. Nos. WO 2016/018960 and WO 2016/018963, each incorporated herein by reference. MERFISH achieves this level of multiplexing by assigning error-robust barcodes to individual RNA species, labeling RNAs combinatorically with oligonucleotide probes that contain a representation of these barcodes, and then reading out these barcodes through sequential rounds of single or multi-color smFISH (Fig. 5). This approach has demonstrated the ability to image ~1000 RNA species in individual cells and profile gene expression in tens of thousands of cells in a single-day-long measurement.

smFISH measurements typically benefit from high signal-to-background ratios, resulting in the detection of individual RNA molecules with high accuracy and detection efficiency. In many cases, the bright fluorescent signals that arise from the tens of fluorescently labeled probes bound to each copy of an RNA far exceed the background that arises from probes binding off target or from cellular autofluorescence. However, as the degree of multiplexing is increased, the background level also tends to increase. The resulting decrease in the signal-to-background ratio challenges many applications and extensions of multiplexed smFISH. For example, many RNAs are not long enough to accommodate tens of oligonucleotide probes, limiting the ability to measure relatively short RNAs and to discriminate many different RNA isoforms. In addition, efforts to further increase the degree of multiplexing, to thousands or potentially tens of thousands of RNAs, will likely be limited by increased background. Finally, background is typically more pronounced in complex tissues, challenging the application of multiplexed smFISH to gene expression profiling in tissue.

This example illustrates a sample clearing approach aimed at improving the signal-to-background ratio in smFISH-based measurements by substantially reducing background fluorescence signal. Many of the modern tissue clearing approaches reduce scattering and autofluorescence background by extracting lipids and matching refractive index while preserving the protein content of the sample. For example, embedding and crosslinking tissues to hydrogels provides a powerful approach to such tissue clearing which preserves cellular proteins and can be made compatible with RNA FISH. However, a major source of background in FISH is the non-specific binding of FISH probes to components other than RNAs in cells. Thus, the examples below illustrate techniques to remove proteins and lipids while preserving RNAs. RNA molecules are anchored to an inert, non-fluorescent polyacrylamide (PA) matrix, effectively imprinting the desired RNA signal on this PA matrix, while clearing the sample of the unwanted, non-RNA components, such as proteins and lipids, thereby removing their contribution to background. These examples demonstrate that this matrix imprinting and clearing approach substantially reduces the background due to off-target binding of FISH probes and autofluorescence. By comparing the copy number of 130 RNAs measured via MERFISH in uncleared and cleared cultures of human cells, it is demonstrated that this matrix-imprinting-based clearing approach improves the detection efficiency and detection limit of MERFISH with no detectable loss in RNAs. Moreover, the reduction in autofluorescence allowed MERFISH imaging to be extended to four distinct color channels with no reduction in performance. This improvement substantially reduced the number of hybridization rounds needed for MERFISH measurements, which should increase the MERFISH measurement speed and throughput. These examples also demonstrate that this clearing approach substantially reduces the background in tissue, facilitating spatial profiling of the expression for 130 genes in cryosections of adult mouse brain tissues, as an example. Imprinting the desired signal, either protein or, more recently, RNA, on a solvent-expandable PA matrix has also been used to physically magnify samples in expansion microscopy; thus, it is expected that the combination of expansion microscopy and RNA FISH may also benefit from the reduction in background demonstrated here. Given the simplicity and efficacy of this matrix-imprinting-based clearing method, it is expected that this approach can be used to substantially improve the performance of a wide range of *in* situ-hybridization methods for both RNA and DNA in both cell culture and tissue, including complex tissues such as the brain.

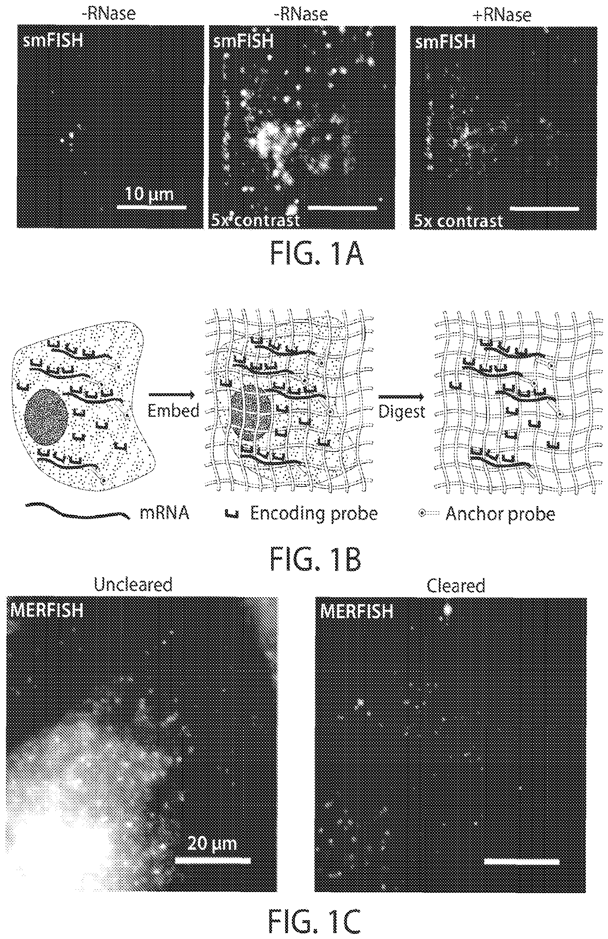

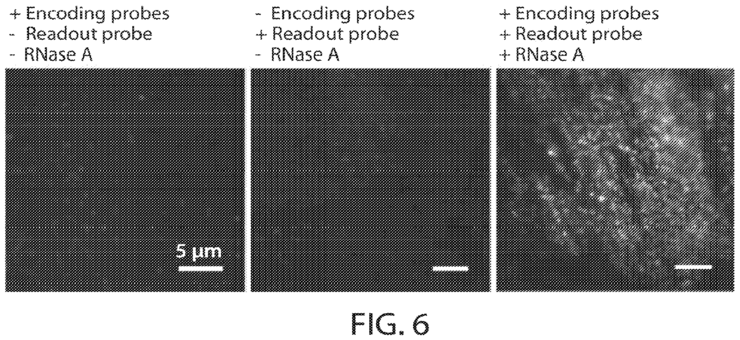

The first step in the development of a sample clearing method for smFISH was to determine the physical origin of off-target binding of oligonucleotide probes: are these probes binding to the incorrect RNA, or other cellular components such as proteins or lipids? To address this question, human lung fibroblast (IMR-90) cells were stained using FISH probes targeting the Filamin A (FLNA) mRNA. As expected, both bright fluorescence spots marking individual molecules of FLNA mRNA (Fig. 1A, left) and a diffuse background due to off-target probe binding (Fig. 1A, middle) were observed that was not present in samples not stained with FISH probes (Fig. 6). The RNase sensitivity of both the foreground RNA spots and the diffuse background was measured. It was reasoned that if the background arises from off-target binding to RNA, both the foreground spots and background should be RNase sensitive.

It was found that a brief RNase A treatment completely removed the bright foreground spots, but produced little if any reduction in the background (Fig. 1A, right). Thus, it was concluded that the vast majority of off-targeting binding of smFISH probes arose from binding to cellular components other than RNA, such as proteins and lipids.

Since this background arises from binding of FISH probes to cellular components other than RNA, it was believed that one way to reduce it would be to remove unwanted components, such as proteins and lipids, from the sample. Moreover, since these components are also a major source of autofluorescence, the autofluorescence background might be reduced by such an approach as well. To this end, the sample was fixed and hybridized with oligonucleotide probes as in a standard smFISH or MERFISH measurement, and then the sample was embedded in an inert, non-fluorescence matrix to which RNAs were anchored, effectively imprinting the desired RNA signal onto this matrix. Once RNAs were anchored, cellular proteins and lipids were removed without, in principle, affecting the number and localization of RNAs within the sample. smFISH probes bound off-target to these components were then free to diffuse from the matrix. Polyacrylamide (PA) was utilized as the inert matrix and a 15-nt-long poly-dT oligonucleotide was used to bind and anchor polyadenylated (polyA) RNAs to the PA matrix. This anchor probe was comprised of 50% locked-nucleic acid bases to stabilize the hybridization to polyA tails of the RNAs and additionally contained a terminal acrydite moiety which could be covalently incorporated into the PA matrix as it polymerizes.

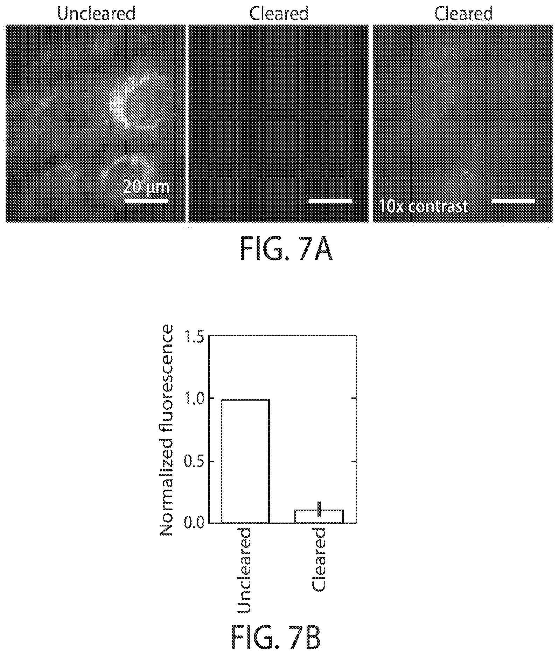

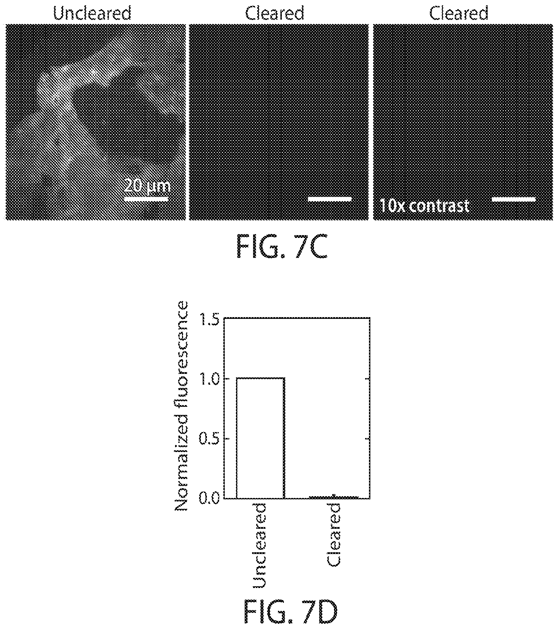

To test whether this clearing approach led to a reduction in off-target binding, the efficacy of protein and lipid removal was first measured. Fig. 7 illustrates that this protocol efficiently removed cellular proteins and lipids from embedded cultured human osteosarcoma cells (U-2 OS). Next, labeling was performed as in MERFISH experiments and whether off-target probe binding was indeed reduced by clearing was tested. U-2 OS cultures were stained with a complex library of "encoding" oligonucleotide probes used for a MERFISH measurement of 130 RNAs. These encoding probes were not themselves fluorescently labeled. Instead, each encoding probe contained a targeting sequence that directed its binding to a cellular RNA and multiple readout sequences, and the collection of encoding probes targeted to each RNA species contained a set of readout sequences that form a specific barcode that is unique to that RNA species. These barcodes were then measured in a series of hybridizations, each with a fluorescently labeled oligonucleotide "readout" probe complementary to a specific readout sequence (see Figs. 5B and 5C). To demonstrate the clearing efficacy, the sample was stained with a total concentration (300 micromolar) of encoding probes that was 3-fold higher than typically used in MERFISH experiments to generate high background. The sample was embedded and cleared in the PA matrix as described above, and then the RNA-imprinted matrix was stained with a readout probe labeled with Cy5 dye (see below). Fig. 5C shows that the cleared samples contained visible smFISH spots but substantially lower background than uncleared samples, demonstrating that this approach indeed reduced the background due to off-target probe binding.

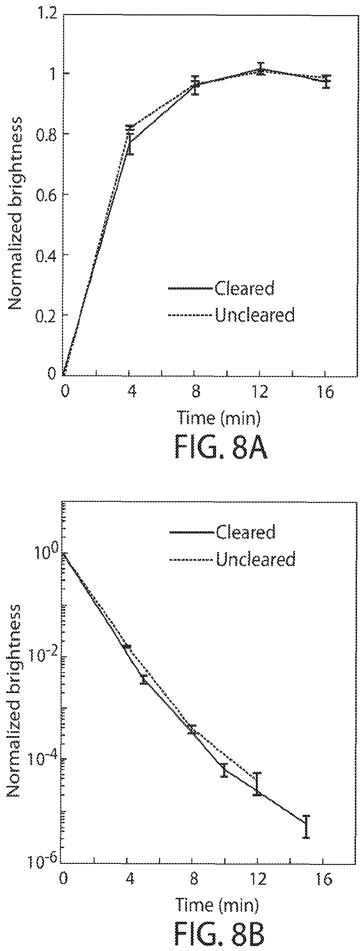

Some MERFISH measurements require repeated sample staining with a series of readout probes, the exchange of a variety of buffers, and, in cases where the FISH signal is removed between consecutive imaging rounds by chemical cleaveage of the fluorophores, the efficient removal of cleaved fluorophores. To facilitate the rapid penetration of buffers and readout probes as well as the rapid removal of cleaved dyes, samples were embedded in 50-100-micrometer-thick PA films (see below). These films were thick enough to cover cultured cells or moderately sized tissue slices, yet thin enough that the rates of readout probe hybridization and of dye cleavage/removal were not substantially changed from those observed in uncleared samples (Fig. 8).

Fig. 1 illustrates matrix imprinting and clearing reduces background in smFISH measurements. Fig. 1A shows a human fibroblast cell (IMR-90) stained with smFISH probes targeting the FLNA mRNA before (left and middle) and after (right) treatment with RNase A. The contrast of the middle and right panels has been increased 5-fold from that of the left panel to better visualize the background from probes bound off-target. Scale bars: 10 micrometers. Fig. 1B shows a schematic diagram of a matrix-imprinting and clearing approach to reduce background in smFISH measurements. Cells were stained with smFISH probes or encoding probes for MERFISH measurements, and a poly-dT anchor probe which targets the polyA tail of mRNAs. Cells were then embedded in a polyacrylamide (PA) matrix, to which the anchor probes were covalently linked via a terminal acrydite moiety. Proteins and lipids were then digested and extracted, freeing off-target bound smFISH probes to diffuse out of the PA matrix and removing cellular components that contribute to autofluorescence. Fig. 1C illustrates U-2 OS cells labeled with MERFISH encoding probes targeting 130 RNAs, uncleared (left) or cleared (right), before staining with a readout probe conjugated to Cy5 that binds to the encoding probes. Scale bars: 20 micrometers.

Fig. 5 shows a schematic diagram of multiplexed error robust fluorescence *in situ* hybridization (MERFISH). Fig. 5A is an illustration of a barcoding process used by MERFISH to identify RNAs. In this implementation of MERFISH, each individual RNA species is assigned a unique binary barcode. This barcode is then read out through a series of smFISH staining and imaging rounds. Each smFISH image is associated with a specific bit in the binary barcode, and only a subset of the targeted RNAs is labeled such that they will fluoresce in each image. If an RNA fluoresces in a given image, then it is assigned a "1" in the corresponding bit. If it does not, then it is assigned a "0." In this fashion, the specific on/off pattern of fluorescence across N smFISH images is used to construct a binary barcode for each measured RNA in the sample, which is then used to decode the identity of that RNA, e.g. A, B, C. Fig. 5B is a schematic depiction of the design of MERFISH probes used here. Individual RNAs are labeled with multiple "encoding" oligonucleotide probes. These encoding probes contain a central target region that has a sequence complementary to a portion of the RNA to which it is targeted. This sequence is flanked by multiple readout sequences. These readout sequences are custom-designed, 20-nt sequences, and there is one unique readout sequence associated with each bit in the barcodes. If an RNA species is assigned a barcode with a "1" in a given bit, then the readout sequence associated with that bit will be contained within the encoding probes that target that RNA; thus, the set of readout sequences associated with each RNA define its barcode. In the MHD4 code used in this work to encode RNAs, each valid 16-bit barcode contains only four "1" bits and hence the set of encoding probes targeting each RNA together contain four different readout sequences. To limit the length of the encoding probes, three of the four readout sequences were randomly to be associated with each encoding probe. After staining the RNAs with encoding probes, the barcodes associated with the RNAs are then measured by a series of hybridizations with fluorescently labeled "readout" probes, each complementary to a readout sequence. Fig. 5C is a schematic depiction of the MERFISH readout process used here. During each round of readout hybridization, one or more readout probes are bound to the sample. Multiple readout probes can be hybridized to the sample simultaneously if each is conjugated to a spectrally distinct dye (different shaded circles). The sample is imaged in all appropriate color channels and the presence or absence of a fluorescent spot determines if the corresponding readout sequence is present and, thus, if the barcode associated with each RNA copy has a "1" or a "0" in the corresponding bit. To remove the fluorescent signal before the next round of smFISH hybridization and imaging, a disulfide bond linking the fluorophores to the readout probes is reductively cleaved and the free fluorophores washed away. The sample is then restained with a different set of readout probes and the process repeated in order to read out the remaining bits in the barcodes.

Fig. 6 illustrates that off-target binding of FISH probes is largely insensitive to RNase treatment. Images of different background sources in IMR-90 cells: Cells stained with encoding probes but no fluorescently labeled readout probe (left), cells stained with a fluorescently labeled readout probe but no encoding probes (middle), and cells stained with encoding probes, a fluorescently labeled readout probe that can bind to a readout sequence on these probes, and then treated with RNase A in order to remove all specific RNA signals (right). All three images are displayed at the same contrast to illustrate the relative intensity of the signal from the autofluorescence background of the cell (left), the very low level (if any) of non-specific binding of readout probes, and the signal from the off-target (RNase-insensitive) binding of encoding probes and readout probes. The encoding probes used here targets the FLNA mRNA only, and the readout probe used here was the Bit-1 readout probe conjugated to Cy5 (Table 1). Scale bars: 5 micrometers.

Fig. 7 illustrates that protease digestion and detergent treatment efficiently remove protein and lipid from polyacrylamide embedded cells. Fig. 7A illustrates images of U-2 OS cells stained with Krypton, a non-specific protein dye, in samples that were either uncleared of cleared. The contrast at which the right image is displayed has been increased 10x relative to the middle image to better illustrate the reduction in fluorescence signal. Fig. 7B shows the average fluorescence signal observed from the samples in Fig. 7A. The average fluorescence has been normalized to the fluorescence observed in the uncleared sample. The error bar represents SEM across three biological replicates. Fig. 7C is similar to Fig. 7A but for DiD, a non-specific lipid stain. Fig. 7D is similar to Fig. 7B but for the samples stained with DiD. Scale bars: 20 micrometers.

Fig. 8 illustrates that matrix imprinting and clearing in PA films does not reduce the rate of readout probe binding or reductive cleavage of fluorescent dyes. Fig. 8A illustrates the average brightness of individual RNA spots as a function of time exposed to a readout probe conjugated to Cy5 in uncleared samples or cleared samples. The average brightness was normalized to the average of the brightness observed in the final two time points. Fig. 8B shows the average brightness of individual RNA spots as a function of time exposed to cleavage buffer. The average brightness has been normalized to that observed prior to exposure to cleavage buffer. Both measurements were conducted on IMR-90 cells stained with encoding probes targeting the FLNA mRNA and the first readout probe (Bit 1; Table 1). The readout hybridization buffer utilized in Fig. 8A differed slightly from that described previously in that it contained 3 nM of the readout probe and no dextran sulfate. All error bars represent SEM across three biological replicates.

Table 1 shows readout probe sequences. The sequences, from top to bottom, correspond to SEQ ID NOs: 1-16. The dye was attached to each readout probe via a disulfide bond at the 3' end of the listed probe sequence.

*Table 1*

| Bit | Readout probe name | Sequence | Dye (2-color MERFISH) | Dye (4-color MERFISH) |
|---|---|---|---|---|
| 1 | RS0015 | ATCCTCCTTCAATACATCCC | Cy5 | Cy5 |
| 2 | RS0083 | ACACTACCACCATTTCCTAT | Alexa750 | Alexa750 |
| 3 | R50095 | ACTCCACTACTACTCACTCT | Alexa750 | ATTO565 |
| 4 | RS0109 | ACCCTCTAACTTCCATCACA | Cy5 | Alexa488 |

(continued)

| Bit | Readout probe name | Sequence | Dye (2-color MERFISH) | Dye (4-color MERFISH) |
|---|---|---|---|---|
| 5 | RS0175 | ACCACAACCCATTCCTTTCA | Cy5 | Cy5 |
| 6 | RS0237 | TTTCTACCACTAATCAACCC | Alexa750 | Alexa750 |
| 7 | RS0247 | ACCCTTTACAAACACACCCT | Cy5 | Alexa488 |
| 8 | RS0255 | TCCTATTCTCAACCT.AACCT | Alexa750 | ATTO565 |
| 9 | RS0307 | TATCCTTCAATCCCTCCACA | A!exa750 | Alexa750 |
| 10 | RS0332 | ACATTACACCTCATTCTCCC | Cy5 | Cy5 |
| 11 | RS0343 | TTTACTCCCTACACCTCCAA | Cy5 | ATTO565 |
| 12 | RS0384 | TTCTCCCTCTATCAACTCTA | A!exa750 | Alexa488 |
| 13 | RS0406 | ACCCTTACTACTACATCATC | Cy5 | Cy5 |
| 14 | RS0451 | TCCTAACAACCAACTACTCC | Alexa750 | Alexa750 |
| 15 | RS0468 | TCTATCATTACCCTCCTCCT | Alexa750 | ATTO565 |
| 16 | RS0548 | TATTCACCTTACAAACCCTC | Cy5 | Alexa488 |

## EXAMPLE 2

This example illustrates that RNA may be preserved during clearing. To determine if any RNAs were lost during matrix imprinting and sample clearing, MERFISH was used to determine the copy number of 130 RNAs in a cleared sample of U-2 OS cells and these numbers to that derived previously from an uncleared sample. A previously published 16-bit, modified Hamming distance-4 (MHD4) code was used to encode RNAs. In this encoding scheme, all valid binary barcodes used to encode RNAs were separated by a Hamming distance of at least 4, which means that at least four bits must be read incorrectly to change one valid barcode to another, drastically reducing the probability of mis-identifying RNAs. Furthermore, this scheme also allowed correction of single-bit errors because every single-bit error produces a barcode uniquely close to a single valid barcode. This specific MHD4 code contained 140 valid barcodes, but only 130 of them were utilized to encode RNAs, leaving the remaining 10 barcodes to serve as "blank" controls to determine the rate of spurious RNA detection and estimate misidentification rates.

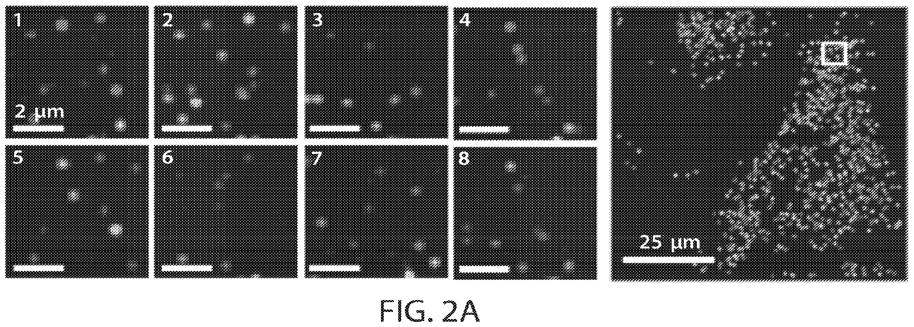

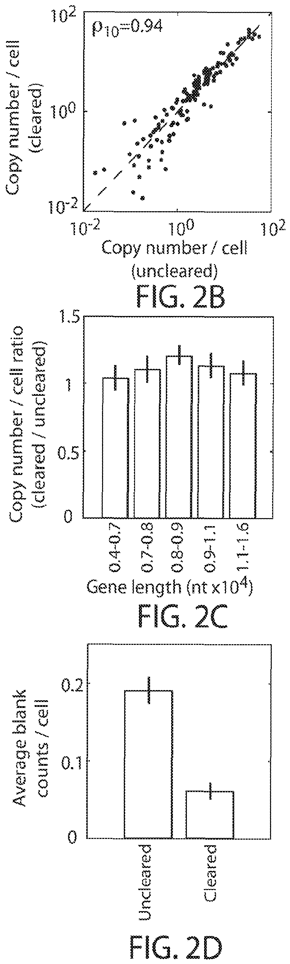

The MERFISH measurements were performed as described previously (see, e.g., Int. Pat. Apl. Pub. Nos. WO 2016/018960 and WO 2016/018963), using two-color imaging to read out 16 bits in 8 rounds of hybridization and imaging as well as reductive cleavage of disulfide bonds to remove the fluorophores linked to the readout probes between consecutive rounds of smFISH imaging (Fig. 5C). Further discussion is provided in Example 5. Fig. 2A shows that individual RNA molecules could be clearly detected in each of the 8 hybridization and imaging rounds. Moreover, Fig. 2B shows that the copy number observed for these 130 RNAs measured in this cleared sample correlated strongly with those measured in a uncleared sample with a Pearson correlation coefficient of 0.94 between the log10 copy numbers (p10, rho-10 = 0.94). Here, and for all following analysis, only the RNAs with copy numbers larger than that observed for the largest "blank" barcode were conservatively utilized. On average, the ratio between the copy numbers measured in the cleared sample to those measured in the uncleared sample was 1.12 +/- 0.04 (SEM, across the 116 RNAs with copy numbers larger than that of the maximum observed for the "blank" barcodes). In addition, this ratio did not have a dependence on the length of the RNA (Fig. 2C).

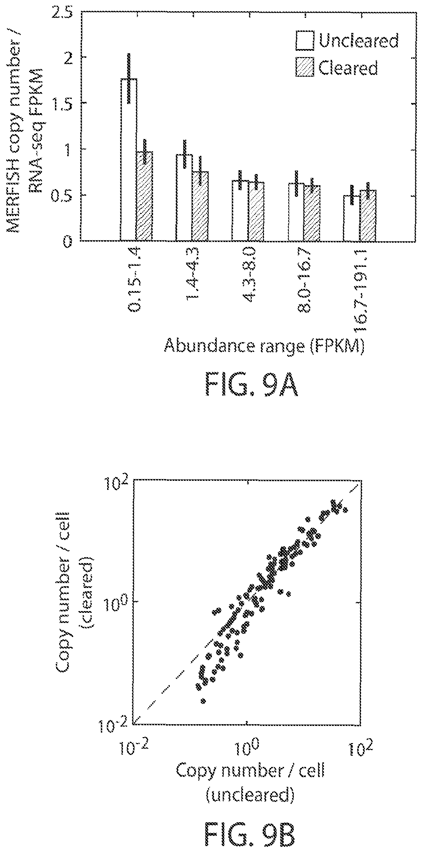

These measurements showed that several aspects of MERFISH performance were improved with matrix imprinting and clearing. A MERFISH detection efficiency of -90% was previously observed in uncleared samples; thus, a copy number ratio of -1.1 between the cleared and uncleared samples suggested that clearing increased this detection efficiency to near 100%. It was also observed that the average frequency at which the "blank" barcodes were observed in the cleared samples dropped substantially relative to that observed in the uncleared samples (Fig. 2D). The average level of the "blank" barcode counts observed in the uncleared sample (Fig. 2D) was comparable to the observed copy number for the lowest abundance RNAs measured, leading to the possibility that the copy number observed for these low abundance RNAs might have been biased by a background rate of spurious RNA counts. Indeed, in uncleared samples, an excess of these low abundance RNAs relative to that expected from bulk RNA-seq was observed (Fig. 9A), whereas this bias was substantially reduced in cleared samples (Fig. 9A and 9B), consistent with the decreased rate of "blank" barcode detection in cleared samples (Fig. 2D). Thus, it was concluded that the increased signal-to-background evident in cleared samples resulted in an improvement in both the detection efficiency and the detection limit in MERFISH measurements.

Fig. 2 illustrates that matrix imprinting and clearing improves MERFISH performance with no loss in RNA. Fig. 2A, left, shows a two-color smFISH images from each of 8 rounds of hybridization and imaging in a 130-RNA MERFISH measurement in matrix imprinted and cleared U-2 OS cells utilizing readout probes labeled with Cy5 or Alexa750. Only a small portion of the MERFISH field of view is shown. Scale bars: 2 micrometers. Right: All identified RNAs detected in a single field-of-view with the identity of the RNA represented by the shading of the marker. The white box represents the portion of this field-of-view displayed in the left panels. Scale bar: 25 micrometers. Fig. 2B shows the average copy number per cell observed for each RNA in U-2 OS cells that were cleared versus that from previously published measurements in an uncleared sample. Copy numbers were corrected by subtracting the average copy number observed for the "blank" barcodes. Fig. 2D shows uncorrected copy numbers displayed in Fig. 9B. The log10 counts correlate with a Pearson correlation coefficient of 0.94 (p-value: $10^{-54}$). The dashed line represents equality. Fig. 2C shows the average ratio of the copy number per cell for a sample that was cleared to that observed for an uncleared sample for RNAs within the specified RNA length range. Error bars represent SEM (N = 26 for each bin). Fig. 2D shows the average copy number per cell of the "blank" barcodes, i.e. barcodes not assigned to an RNA, in an uncleared sample and a cleared sample. Error bars represent SEM across all 10 "blank" barcodes.

Fig. 9 shows that matrix imprinting and clearing reduces bias in the detection of low abundance RNAs. Fig. 9A shows the ratio of the copy number per cell as determined via MERFISH to the abundance as determined via RNA-seq as measured in FPKM for uncleared samples and cleared samples. Error bars represent SEM across the RNAs in each RNA-seq abundance range (N = 26). Fig. 9B shows the copy number per cell as determined via MERFISH in a cleared sample as compared to that determined for an uncleared sample. These copy numbers have not been corrected for the average rate of "blank" barcode detection as in Fig. 2B. The dashed line represents equality. The deviation from equality in Fig. 2B and the excess MERFISH counts relative to those estimated from bulk-seq at the low abundance range are consistent with the increased rate of 'blank' barcode detection observed for untreated samples (Fig. 2D).

## EXAMPLE 3

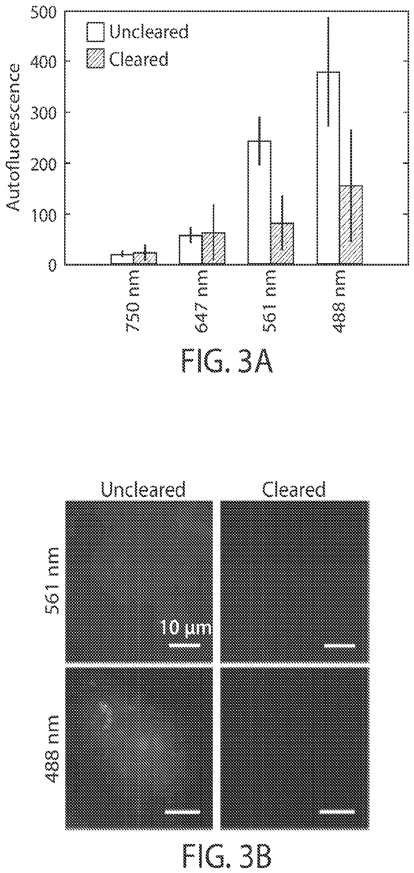

The example illustrates the extension of MERFISH to four-color imaging. In addition to providing a substantial decrease in the background due to off-target binding of FISH probes, the removal of protein and lipid from the sample may also reduce the level of autofluorescence. To quantify this decrease, the fluorescence of unlabeled U-2 OS cells in uncleared and cleared samples was measured at four excitation wavelengths: 750 nm, 647 nm, 561 nm, and 488 nm. Consistent with the expectation that cell autofluorescence is substantially higher in the blue-green spectral range than in the red range, the clearing protocol had little effect on the already low autofluorescence background in the 750-nm and 647-nm channels, but produced a several-fold reduction in the autofluorescence observed in the 561-nm and 488-nm channels (Fig. 3A and B). Additional details may be found in Example 5.

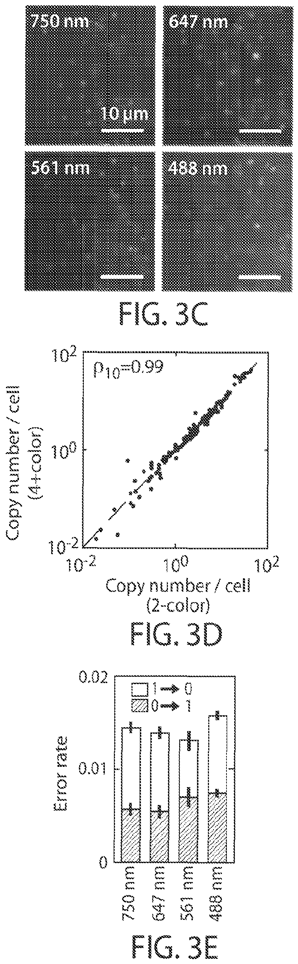

With the significant reduction in the autofluorescence observed in the 561-nm and 488-nm excitation channels, the possibility of using all four excitation channels to read out four different bits simultaneously in each round of imaging during MERFISH measurements was studied. U-2 OS cells were stained with the same MERFISH encoding probe set as described above and MERFISH measurements were performed in which each round of hybridization utilized four different readout probes, conjugated respectively to Alexa750, Cy5, ATTO565, or Alexa488 via a disulfide bond (Table 1). With four colors of readout probes, the full 16-bit MERFISH measurement only required four rounds of hybridization and imaging. The measured copy numbers derived from this four-color measurement were compared to those determined with two-colors in the cleared sample. Fig. 3D demonstrates that these copy numbers correlated strongly with a $\rho10$ (rho-10) of 0.99 and had an average ratio of 1.01 +/- 0.02 (SEM, across the 109 genes with copy numbers above that of the maximum observed for the "blank" barcodes). To confirm that imaging in the new color channels did not introduce additional error, the "1" to "0" or "0" to "1" error rates per bit were determined. It was found that these error rates did not vary substantially with the color channel (Fig. 3E).

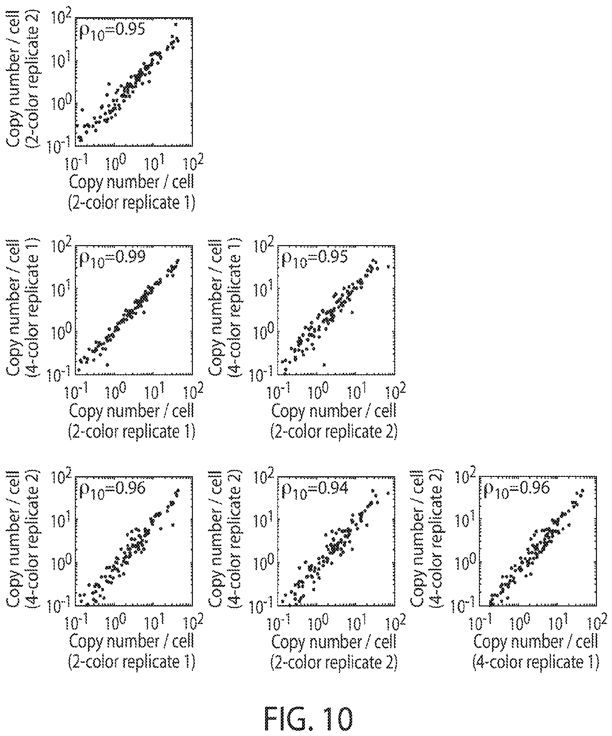

Finally, to confirm that the improved performance with the cleared samples was reproducible, additional two-color and four-color MERFISH measurements were performed in cleared samples. Fig. 10 shows that the copy numbers derived from all of these measurements correlated strongly (all $\rho10$, rho-10, were 0.94 or greater). By comparing each of these data sets to uncleared measurements, an average MERFISH detection efficiency of 96 +/- 7% was estimated (SEM over four replicate measurements) and a ~4-fold reduction in the average rate of "blank" barcode detection (0.08 +/- 0.03 per cell (SEM over four replicate measurements) versus 0.30 +/- 0.07 per cell (SEM over seven previously published replicate measurements)) for cleared and uncleared samples), confirming that clearing improved the detection efficiency and detection limit of MERFISH.

Fig. 3 shows autofluorescence reduction facilitates four-color MERFISH. Fig. 3A shows the average autofluorescence observed for unstained U-2 OS cells before and after matrix imprinting and clearing when excited with 750-nm, 647-nm, 561-nm, or 488-nm light. Error bars represent SEM over three biological replicates. Fig. 3B shows images of unstained U-2 OS cells that were uncleared or cleared excited with either 561-nm or 488-nm light. Fig. 3C shows images of cleared U-2 OS cells stained with a 130-RNA, 16-bit MERFISH encoding probe set and the first four readout probes each conjugated to one of the following dyes: Alexa750, Cy5, ATTO565, or Alexa488. Samples were imaged with excitation light listed in Fig. 3A. Scale bars: 10 micrometers. Fig. 3D shows average copy number per cell determined via four-color MERFISH to that determined with two-color MERFISH, both in cleared samples. The copy numbers have been corrected by subtracting the average rate of "blank" barcode detection as in Fig. 2B. The dashed line represents equality. The Pearson correlation coefficient between the log10 abundances is 0.99 (p-value: $10^{-98}$. Fig. 3E shows the average rate of observing a "1" to "0" error or a "0" to "1" error per bit for bits that are read out with each of the four different fluorophores, as indicated by the excitation wavelength. Each error rate ("1" to "0" or "0" to "1") was calculated for each individual bit using the frequency at which errors were corrected at that bit, and then these per-bit error rates were averaged for bits that used the same fluorophore for measurement (Table 1). Error bars represent SEM over the four bits read out with each dye.

Fig. 10 shows that two- and four-color MERFISH measurements in matrix imprinted and cleared samples are reproducible. Comparison of the average copy number per cell measured in different two-color or four-color MERFISH measurements in cleared U-2 OS cells. $\rho$10 (rho-10) represents the Pearson correlation coefficient between the log10 copy numbers for all RNAs. The p-values associated with all $\rho$10 (rho-10) are less than $10^{-44}$.

**EXAMPLE 4**

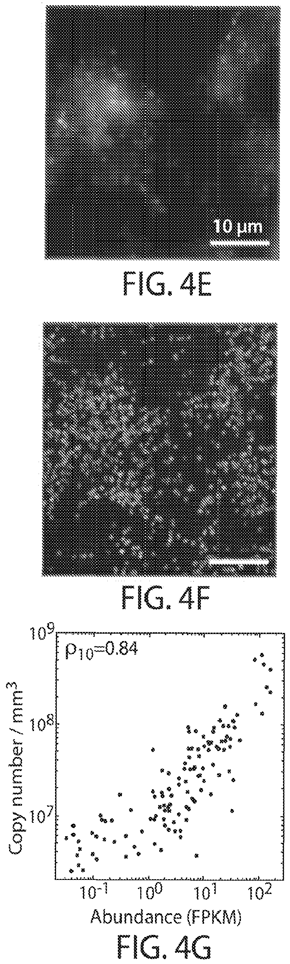

This example illustrates MERFISH measurements of brain tissue. To explore whether clearing can overcome the increased background that has been observed in tissue, MERFISH measurements were performed of 130 RNA species on ~2-mm $\times$ 2-mm, 10-micrometer-thick cryosections taken from adult mouse hypothalamus (Fig. 4A and B). These RNAs were encoded with a 16-bit MHD4 code and read out with 8 rounds of hybridization using two-color imaging per round. See, e.g., Int. Pat. Apl. Pub. No. WO 2016/018960 or WO 2016/018963, each incorporated herein by reference in its entirety. These samples were cleared as described above but with the addition of a brief treatment with 4% w/v sodium dodecyl sulfate (SDS) prior to PA embedding, which further improved clearing in tissue (see below). Fig. 4C and D illustrate that this clearing approach substantially reduced the background observed in these tissue slices, and smFISH spots representing individual RNA molecules were clearly observable in the cleared sample in each round of imaging, allowing individual RNAs to be decoded (Fig. 4E and F). See also Example 5 for additional details.

To determine the accuracy of these measurements, the average RNA density determined via MERFISH for four such tissue slices was compared with the abundance determined via bulk RNA-seq data derived from the same region of the hypothalamus (Fig. 4G). It was found that these values correlated strongly ($\rho$10, rho-10 = 0.84). At very low abundance-corresponding to RNAs that are expressed very poorly in the hypothalamus (< 0.5-1 FPKM)-it was observed that MERFISH copy number did not correlate strongly with that estimated from bulk-sequencing, suggesting that the abundance of these RNAs was below the detection limit, a conclusion supported by the similarity between these copy numbers and the average copy number observed for the blank barcodes ($6\times10^6$ +/- $2\times10^6$ /mm$^3$).

Massively multiplexed smFISH allows spatially resolved gene expression profiling within single cells. However, many applications of and advances to this approach are challenged by the fluorescence background encountered in these experiments. These examples describe a clearing approach that substantially reduced several background sources in FISH measurements by effectively imprinting the desired RNA signal onto an inert, non-fluorescent, PA matrix and then removing unwanted cellular components that give rise to autofluorescence and background due to off-target probe binding. The reduction in background led to improvement in the detection efficiency and detection limit in MERFISH measurements. Moreover, the reduction in autofluorescence in the blue and green color channels allowed MERFISH measurements to be extended to four colors with no loss in performance. This advance in turn allowed MERFISH measurements with substantially fewer rounds of hybridization and imaging, which can increase the MERFISH measurement speed and throughput. Finally, matrix imprinting and clearing produced a substantial reduction in the background observed for MERFISH measurements in tissue samples, allowing gene expression for 130 RNAs in cryosections of the mouse hypothalamus to be characterized.

Substantial reduction in background provided by this clearing approach will facilitate future extensions of MERFISH. An increase in the degree of multiplexing-to the simultaneous measurement of thousands or tens of thousands of RNAs-would likely require far higher encoding probe concentrations than are currently used and, thus, will benefit from the much lower off-target probe binding achieved in cleared samples. With lower background, it should be possible to detect RNAs with fewer numbers of bound probes, which should in turn allow shorter RNA molecules to be detected. This may facilitate the detection of relatively short messenger and long-non-coding RNAs, and even possibly small RNAs, which are currently difficult to detect in uncleared samples. The ability to detect RNA molecules with relatively few FISH probes will also substantially improve the ability to distinguish RNA isoforms. The combination of expansion microscopy with MERFISH, facilitated by a common matrix imprinting approach, may also allow a higher density of RNA molecules to be resolved with MERFISH-an ability that could be useful for measuring dense regions of highly expressed RNAs and for further increasing the degree of multiplexing. Thus, this approach based on matrix imprinting will substantially enhance the ability to perform spatially resolved gene expression profiling with massively multiplexed FISH.

Fig. 4 shows MERFISH measurements of adult mouse brain tissue. Fig. 4A shows Nissl-stained images of coronal and sagittal slices of an adult mouse brain taken from the Allen brain atlas. The black box and dashed line represent the region of the mouse hypothalamus studied. Scale bar: 2 mm. Fig. 4B shows an image of a single, 10-micrometer-thick cryosection of the mouse hypothalamus stained with DAPI (4',6-diamidino-2-phenylindole, dihydrochloride). The entire slice was imaged via MERFISH. Scale bar: 1 mm. Figs. 4C and 4D shows images of a small portion of a mouse hypothalamus slice stained with an encoding probe set for a 130-RNA MERFISH measurement and a readout probe conjugated to Cy5 in a sample. Fig. 4C is an image of an uncleared sample; Fig. 4D is an image of a cleared sample. Scale bar: 50 micrometers. Fig. 4E shows a zoom-in of the region of Fig. 4D marked with the white dashed box. Fig. 4F shows decoded RNAs (different shadings represent different barcodes) for the region shown in Fig. 4E. Fig. 4G shows the density of 130 different RNAs as determined via MERFISH versus the abundance as determined via bulk RNA-seq for the region of the mouse hypothalamus shown in Fig. 4A. The Pearson correlation coefficient between the log10 abundances is 0.84 (p-value: $10^{-35}$).

## EXAMPLE 5

The following describes various materials and methods used in the above examples.

Human osteosarcoma cells (U-S OS, American Type Culture Collection) and human fibroblasts (IMR-90, American Type Culture Collection) were cultured, fixed, permeabilized, and stained with smFISH probes or MERFISH encoding probes as described previously See, e.g., Chen, K.H., et al., "Spatially resolved, highly multiplexed RNA profiling in single cells," Science, 348(6233):aaa6090 or Moffitt, J.R., et al., "High-throughput single-cell gene-expression profiling with multiplexed error-robust fluorescence in site hybridization," Proc. Natl. Acad. Sci. USA, 113(39):11046-11051. Mouse hypothalamus tissue was freshly frozen, cryosectioned into 10-micrometer-thick slices, post-fixed onto coverslips, cleared with 4% w/v SDS, permeabilized with 70% ethanol, and then stained with encoding probes. Cells or tissue samples were embedded in a 4% solution of a 19:1 ratio of acrylamide to bisacrylamide containing 50 mM Tris HCl (pH 8), 300 mM NaCl, 0.03% w/v ammonium persulfate, and 0.15% v/v TEMED. Protein and lipids were removed with a -16 hour, 37 °C digestion with proteinase K in 0.8 M guanidine HCl, 0.5% v/v Triton X-100, 50 mM Tris pH 8, and 1 mM EDTA.

MERFISH measurements with U-2 OS cells were performed with a published encoding probe set. The encoding probe set for measurements in mouse brain tissue was designed as described previously. Readout probes were purchased from Biosynthesis, Inc. and are described in Table 1. Encoding probes were constructed as previously described. See, e.g., Moffitt, J.R., et al., "RNA Imaging with Multiplexed Error-Robust Fluorescence In Situ Hybridization (MERFISH)," Methods Enzymol., 572:1-49, 2016.

Samples were imaged on either a custom platform built around an Olympus IX-71 body, a 1.45 NA, 100x oil-immersion objective, and EMCCD camera or a custom high-throughput platform build around an Olympus IX-71 microscope body, a PlanApo, 1.3 NA, 60x silicone-oil-immersion objective, and scientific CMOS camera. Readout hybridization, buffer exchange, and reductive cleavage were performed with the same buffers and the same automated fluidics system as described previously, with the notable exceptions that dextran sulfate was removed from readout hybridization buffers and the readout probes were stained at a concentration of 3 nM each.

MERFISH measurements in human osteosarcoma cells (American Type Culture Collection, U-2 OS) were performed with the same MERFISH encoding probe set as previously described. Briefly, this encoding scheme utilized a 16-bit Modified Hamming Distance 4 code (MHD4) to encode the RNAs. In this encoding scheme, each of the 140 possible barcodes required at least four errors to accumulate to be converted into another barcode. This property permitted the detection of errors at up to any two bits, and the correction of errors to any single bit. In addition, this encoding scheme utilized a constant Hamming weight, i.e. the number of "1" bits in each barcode, of 4, in order to minimize potential bias in the measurement of different barcodes due to a differential rate of "1" to "0" and "0" to "1" errors. 130 of the 140 possible barcodes were used to encode cellular RNAs, and the remaining 10 barcodes were left unassigned to serve as "blank" controls. The encoding probe set that was used contained 92 encoding probes per RNA, with each encoding probe containing three of the four readout sequences assigned to each RNA (Fig. 5B).

The MERFISH encoding probes for measurements in the mouse hypothalamus were designed using the same 16-bit MHD4 code as above. Again, 130 of the 140 possible barcodes were assigned to RNAs that were selected to cover roughly three orders of magnitude in average expression in the hypothalamus with expression levels estimated from a previously published RNA-seq. The remaining 10 barcodes were left unassigned to serve as blank controls. Encoding probes were designed using the same stringency conditions and design criteria as described previously. Transcript sequences were derived from the mouse genome (mm9) downloaded from ensembl.

Construction of the encoding probe sets was conducted from complex oligonucleotide pools, as described previously. See, e.g., Int. Pat. Apl. Pub. No. WO 2016/018963, incorporated herein by reference. Briefly, the oligopools (CustomArray) were amplified via limited-cycle PCR to make *in vitro* transcription templates. These templates were converted into RNA via *in vitro* transcription, the RNA back converted back to DNA via reverse transcription, and then the DNA was purified via alkaline hydrolysis (to remove RNA templates), phenol-chloroform extraction (to remove proteins), and ethanol precipitation (to remove nucleotides and concentrate probes). To improve probe purity and reaction yield, the previous protocols were modified in the following ways. Excess NTPs or dNTPs were removed via desalting columns (40K MWCO Zeba™; ThermoFisher, 89894) after both the *in vitro* transcription and the phenol-chloroform extraction. It was found that removal of stray NTPs improved the performance of the reverse transcription and that removal of excess dNTPs aided in quantification of the final yield of the protocol. In addition, to further improve yield, the salt in the ethanol purification was switched from 2.5 M ammonium acetate (which allows nucleotides to be removed, but decreases DNA recovery) to 300 mM sodium acetate.

To stabilize the polyacrylamide (PA) film, the coverslips were coated with a silane layer containing an allyl moiety which could be actively incorporated into polyacrylamide gels during polymerization, covalently crosslinking the PA film to the coverslip. Briefly, 40-mm-diameter, #1.5 coverslips (Bioptechs, 0420-0323-2) were washed for 30 minutes via immersion in a 1:1 mixture of 37% HCl and methanol at room temperature (RT). The coverslips were then rinsed three times in deionized water and once in 70% ethanol. The coverslips were dried in a 70 °C oven and then immersed in 0.1% v/v triethylamine (Millipore, TX1200) and 0.2% v/v allyltrichlorosilane (Sigma, 107778) in chloroform for 30 minutes at room temperature (RT). The coverslips were washed once each with chloroform and ethanol and then baked in a 70 °C oven for one hour to dehydrate the silane layer. The silanized coverslips could then be stored at room temperature in a desiccated chamber for weeks with no obvious reduction in the quality of the silane layer.

To promote cell adhesion, the silanized coverslips were coated with 0.1 mg/mL poly-D-lysine (PDL) (molecular weight 30,000-70,000 Da; Sigma, P7886) diluted in nuclease-free water for 1 hour at room temperature. The coverslips were washed three times with nuclease-free water, incubated in water at room temperature overnight, and then dried and UV sterilized prior to plating cells. U-2 OS cells were cultured, fixed, and permeabilized using established protocols, before staining with encoding probes. Briefly, cells cultured with Eagle's Minimum Essential Medium (American Type Culture Collection, 30-2003) containing 10% v/v fetal bovine serum (ThermoFisher, 10437) were plated on PDL-coated, silanized coverslips at a density of 300,000 cells per coverslip and incubated at 37 °C with 5% $CO_2$ for 48 to 72 hours before fixing with 4% paraformaldehyde (PFA; Electron Microscopy Sciences, 15714) in 1x phosphate buffered solution (PBS; ThermoFisher, AM9625) for 20 minutes. The cells were washed three times with 1×PBS, and permeabilized using 0.5% v/v Triton X-100 (Sigma, T8787) in 1×PBS for 10 minutes at room temperature. The cells were then washed three times with 1×PBS.

Briefly, cells were incubated for 5 minutes in a 30% formamide wash buffer, containing 2xsaline-sodium citrate (SSC; ThermoFisher, AM9763) and 30% v/v formamide (ThermoFisher, AM9342) and then stained with encoding probes in encoding hybridization buffer, containing 2xSSC, 30% v/v formamide, 0.1% w/v yeast tRNA (Life technologies, 15401-011), 1% v/v murine RNase inhibitor (NEB, M0314L), and 10% w/v dextran sulfate (Sigma, D8906), in a humidity-controlled 37 °C incubator for 36 to 48 hours. Encoding probes were stained at a concentration of 100 micromolar unless otherwise specified. Where appropriate, the encoding probes were supplemented with 1 micromolar of anchor probes- a 15-nt sequence of alternating dT and thymidine-locked nucleic acid (dT+) with a 5'-acrydite modification (Integrated DNA Technologies). After staining, cells were washed two times for 30 minutes each with 30% formamide wash buffer at 47 °C.

Human lung fibroblast cells (American Type Culture Collection, IMR-90) were cultured, fixed, and stained following the same protocols described above for U-2 OS cells using 1 micromolar of a smFISH probe set targeting Filamin A (FLNA, Biosearch).

In order to anchor RNAs in place, the encoding-probe-stained samples were embedded in thin, 4% PA gels. Briefly, stained samples were first washed for two minutes with a de-gassed PA solution, having 4% v/v of 19:1 acrylamide/bis-acrylamide solution (BioRad, 1610144), 60 mM Tris-HCl pH 8 (ThermoFisher, AM9856), 0.3 M NaCl (ThermoFisher, AM9759), and either a 1:500 dilution of 0.1-micrometer-diameter light-yellow beads (Spherotech, FP-0245-2) when samples were used for four-color MERFISH measurements or a 1:200,000 dilution of 0.1-micrometer-diameter carboxylate-modified orange fluorescent beads (Life Technologies, F-8800) when samples were used for two-color MERFISH measurements. The beads served as fiducial markers for the alignment of images taken across multiple rounds of smFISH imaging. Cells were then washed again for 2 minutes with the same PA gel solution supplemented with the polymerizing agents ammonium persulfate (Sigma, A3678) and N,N,N',N'-tetramethylethylenediamine (TEMED; Sigma, T9281) at final concentrations of 0.3% w/v and 0.15% v/v, respectively.

To cast a thin PA film, 50 microliters of this gel solution was added to the surface of a glass plate (TED Pella, 26005) that had been pre-treated so as not to stick to PA (GelSlick, Lonza, 50640). The sample was aspirated to remove excess PA gel solution, then gently inverted onto this 50-microliter droplet to form a thin layer of PA between the coverslip and the glass plate. The volume of this gel droplet could be used to control the thickness of this PA film. The gel was then allowed to cast for 1.5 hours at room temperature. The coverslip and the glass plate were then gently separated, and the PA film washed twice with a digestion buffer with 0.8 M guanidine-HCl (Sigma, G3272), 50 mM Tris-HCl pH 8, 1 mM EDTA, and 0.5% v/v Triton X-100 in nuclease-free water. After the final wash, the gel was covered with digestion buffer supplemented with 1% v/v proteinase K (NEB, P8107S). The sample was digested in this buffer for 16 to 20 hours in a humidified, 37 °C incubator and then washed with 2xSSC three times. MERFISH measurements were either performed immediately or the sample was stored in 2xSSC supplemented with 0.1% v/v murine RNase inhibitor at 4 °C for no longer than 24 hours.

Cultured-cell samples were imaged on a home-built imaging platform. Briefly, this microscope was built using an Olympus IX-71 body and a 1.45 NA, 100x oil-immersion objective. Illumination in 750 nm, 641 nm, 561 nm, and 488 nm were provided using solid-state lasers (MPB communications, VFL-P500-751; MPB communications, VFL-P500-642; Coherent, 561-200CWCDRH; and Coherent, 1069413/AT) for excitation of readout probes labeled with Alexa750, Cy5, ATTO565 and Alexa488, respectively. For two-color MERFISH measurements, the 561-nm laser was used to excite the orange fiducial beads. A 405-nm solid-state laser (Coherent, Cube) was used to illuminate the nuclear stain 4', 6-diamidino-2-phenylindole, dihydrochloride (DAPI), where appropriate, and the light-yellow fiducial beads during four-color MERFISH measurements. All laser lines were combined with a custom dichroic (Chroma, zy405/488/561/647/752RP-UF1), and the emission was filtered with a custom dichroic (Chroma, ZET405/488/561/647-656/752m). Fluorescence was separated with a custom penta-notch filter and imaged with an EMCCD camera (Andor, iXon-897). The pixel size for the EMCCD camera was determined to correspond to 167 nm in the sample plane.

Tissue slices were imaged on a second home-built imaging platform optimized for throughput. Briefly, this microscope was constructed around an Olympus IX-71 microscope body and a PlanApo, 1.3 NA, 60x silicone-oil-immersion objective (Olympus, UPLSAPO 60xS2). Illumination in 754 nm, 647 nm, 561 nm, and 405 nm was provided using solid-state lasers (Toptica, DL100/BoosTA; MBP Communications, F-04306-113; Crystalaser GCL-150-561; Coherent, Cube 405). These laser lines were used to excite readout probes labeled with Alexa750 and Cy5, orange fiducial beads, and DAPI, respectively. The illumination profile was flattened with a square multi-mode fiber (Andor, Borealis). The fluorescence emission from the sample was separated from the laser illumination using a penta-band dichroic (Chroma, zy405/488/561/647/752RP-UF1) and imaged using a scientific CMOS camera (sCMOS; Andor, Zyla 4.2) after passing through two duplicate custom penta-notch filters (Chroma, ZET405/488/561/647-656/752m) to remove stray excitation light. The pixel size for the sCMOS camera was determined to correspond to 109.2 nm in the sample plane. During the imaging of tissue, z-stacks consisting of seven, 1.5-micrometer-slices were collected in each color channel at each field-of-view (FOV) so as to image the entire volume of the tissue. The z-steps were controlled via an objective nanopositioner (Mad City Labs, NanoF200).

On both setups, sample position was controlled via a motorized microscope stage (Marzhauser, SCAN IM 112x74) and focus was maintained via a custom focus-lock system, realized through a feedback system between an objective nanopositioner (Mad City Labs, NanoF200) and the reflection of an IR laser (Thorlabs, LP980-SF15) onto an inexpensive CMOS camera (Thorlabs, uc480). The sample coverslip was held inside a flow chamber (Bioptechs, FCS2), and buffer exchange within this chamber was directed using a custombuilt automated fluidics system), controlling three eight-way valves (Hamilton, MVP and HVXM 8-5) and a peristaltic pump (Gilison, Minipuls 3). The entire system was computer-controlled via custom software.

Samples were hybridized with readout probes and imaged following protocols similar to those previously described, with slight adjustments to readout hybridization buffer composition and flow times. See Int. Pat. Apl. Pub. Nos. WO 2016/018963 and WO 2016/018960, each incorporated herein by reference in its entirety. Readout hybridization buffer was composed of 2xSSC, 10% v/v ethylene carbonate (EC; Sigma-Aldrich, E26258), 0.1% v/v murine RNase inhibitor in nuclease-free water, and 3 nM of the appropriate readout probes. Previously, dextran sulfate was utilized in this buffer to increase the rate of readout probe hybridization; however, in was found that the same hybridization kinetics can be achieved without dextran sulfate by increasing the readout probe concentrations from 1 nM to 3 nM. Removing dextran sulfate from the readout buffer dramatically reduced the viscosity of this buffer, which in turn, effectively eliminated the occasional flow failures that arose from the high pressures required to pull high viscosity buffers through the fluidics system.

Two different configurations of readout probes were utilized: for 2-color MERFISH measurements, two readout probes, one conjugated to Cy5 and the other to Alexa750 via a disulfide bond were used in each round of hybridization; and for 4-color MERFISH four different readout probes each conjugated to one of Alexa750, Cy5, ATTO565, or Alexa488 via a disulfide bond were used in each round of hybridization. Table 1 contains the readout probe sequences and dye combinations used for both two- and four-color measurements. All readout probes were purchased from Biosynthesis, Inc.

The sample was stained with readout probes by first flushing the sample chamber with 2 mL of readout hybridization buffer over the span of 5 minutes to fully exchange buffers. Then an additional 2 mL of buffer was flowed across the sample for 6 minutes. The sample was then washed by flowing 2 mL of readout wash buffer, containing 2xSSC and 10% v/v EC, over a span of 9 minutes. Finally, 2 mL of imaging buffer, containing 2xSSC, 50 mM Tris-HCl pH 8, 10% w/v glucose, 2 mM Trolox (Sigma-Aldrich, 238813), 0.5 mg/mL glucose oxidase (Sigma-Aldrich, G2133), 40 micrograpms/mL catalase (Sigma-Aldrich, C30) and 0.1% v/v murine RNase inhibitor, was flowed across the sample for 6 minutes, after which the flow was halted and ~400 FOVs were imaged. The imaging buffer was stored under a layer of mineral oil (Sigma-Aldrich, 330779) throughout the measurement as a barrier against oxygen. Because glucose oxidase was determined to contain trace amounts of RNase, the imaging buffer also contained 0.1% v/v murine RNase inhibitor. The ribonucleoside vanadyl complex (VRC; NEB, S1402S), which was used previously, was replaced with Murine RNase inhibitor.

After each round of imaging, the fluorescent dyes were removed from readout probes by reductive cleavage of the disulfide bond conjugating these dyes to the probes. 3 mL of cleavage buffer comprising 2xSSC and 50 mM of the reducing agent Tris(2-carboxyethyl)phosphine (TCEP; Sigma, 646547) was flowed across the sample over the course of 15 minutes. After cleavage, the chamber was flushed with 2 mL of 2xSSC for 4 minutes to flush any residual cleavage buffer from the sample prior to the introduction of the subsequent hybridization buffer. All buffers were freshly prepared before each experiment using nuclease-free water.

After the final round of hybridization and imaging, the sample was stained with DAPI at a concentration of 1 microgram/mL in 2xSSC for 10 minutes to mark nuclei, and then imaged at 405 nm.

Registration of images of the same FOV across imaging rounds as well as decoding of the RNA barcodes was conducted using an analysis pipeline. Briefly, the locations of the fiducial beads in each round of imaging were found via a Gaussian fitting routine, and these locations were used to create affine transformations that correct offsets between images in each imaging round. Additional corrections to account for minor chromatic aberrations were not applied because the offsets in the centroid of RNAs labeled simultaneously with Alexa750, Cy5, ATTO565, and Alexa488 were not substantial. Images were then high-pass filtered to remove background, deconvolved to tighten RNA spots, and then low-pass filtered so as to connect RNA centroids that differ slightly in location between images. Individual pixels were then assigned to barcodes by comparing the intensity of each pixel across the 16 images collected across all of the hybridization rounds to each of the different barcodes. Specifically, the set of 16 intensities for each pixel derived from each of the 16 imaging rounds were used to define a vector that was normalized to unitary magnitude, i.e. by dividing by the L2 norm. A unit vector was similarly defined for each of the 140 barcodes. The Euclidean distance was then calculated between each pixel vector and each of the barcode vectors. A pixel was assigned to a barcode if the Euclidean distance separating it from a barcode was smaller than a given threshold. This distance threshold was determined from the largest Euclidean distance between each normalized barcode and the set of normalized barcodes formed from all single-bit errors to that barcode. Conceptually, this distance defines a sphere that contains all possible modifications to a barcode that correspond to a single-bit error to that barcode, and this decoding approach can be thought of as assigning pixels to a given barcode based on whether they fall within this sphere for a given barcode. Pixels with vectors that do not fall within one of these 140 spheres are left unassigned. Contiguous pixels assigned to the same barcode were combined to form a single RNA. Each RNA was then identified as requiring error correction (or not) by comparing the average pixel vector across all pixels assigned to that RNA to the set of unitary vectors defined by all single-bit errors to the assigned barcode. If the average pixel vector was closer to a vector corresponding to a single-bit error than it was to the correct barcode, the RNA was marked as requiring error correction.

This decoding approach assumed that the brightness of each RNA spot is identical between imaging rounds. To correct for differences in the brightness between color channels, images were initially normalized by equalizing their intensity histograms. This normalization was then refined via an iterative process. A background of spurious RNAs were removed with thresholds on the brightness of the RNA, i.e. the L2 norm of the pixel vector, and the number of pixels combined to form that RNA, i.e. its area. For tissue imaging, this pipeline was modified to accommodate z-stacks. Because each z-stack was separated by a distance larger than the axial extent of the point-spread-function, each stack was decoded independently of the others. Nuclei were identified and counted via intensity thresholding of the DAPI images. All software were written in Matlab.

Computations were split between the Odyssey cluster supported by the FAS Division of Science, Research Computing Group at Harvard University and a desktop server, which contained two 10-core Intel Xeon E5-2680 2.8 GHz CPUs and 256 GB of RAM.

U-2 OS or IMR-90 samples were stained with smFISH probes or MERFISH encoding probes as described above. The samples were then stained with readout probe 1 (Table 1) as described above with respect to MERFISH imaging. The samples were imaged, and then treated for 30 minutes with 1% v/v RNase A (Qiagen, 19101) in 2xSSC, and then reimaged.

U-2 OS cells were cultured, fixed, and labeled with smFISH probes or MERFISH encoding probes as described above. Samples were then either matrix imprinted and cleared as described above or stored at 4 °C in 2xSSC. Cells were stained with a 1:10 dilution of Krypton Fluorescent Protein Stain (ThermoFisher, 46629) in 2xSSC at room temperature for 15 minutes and washed once in 2xSSC at room temperature for 15 minutes. 100 FOVs were imaged with the 561-nm laser. Samples for lipid staining were prepared in the same fashion but stained with a 1:200 dilution of Vybrant® DiD Cell-Labeling Solution (ThermoFisher, V22887) in 2xSSC at RT for 15 minutes and washed once briefly with 2xSSC. 100 FOVs were imaged with the 641-nm laser. Imaged samples were quantified by averaging the observed fluorescence across all FOVs, and this value was then averaged across three biological replicates.

MERFISH imaging in tissue was performed on 10-micrometer cryosectioned mouse hypothalamus. Whole brain tissue was removed from mice (C57Bl6/J) euthanized using $CO_2$, and immediately frozen in optimum cutting temperature compound (Tissue-Tek O.C.T.; VWR, 25608-930). Frozen blocks were coarsely sectioned to the hypothalamus region, trimmed to an area of roughly 3 mm × 3 mm, and sectioned at a thickness of 10 micrometers at -18 °C on a cryostat (MICROM, HM550). Sections were collected on silanized coverslips coated with PDL prepared following protocols described above. These sections were then immediately fixed in 4% PFA in 1×PBS for 12 minutes at RT and washed with 1×PBS for 5 minutes three times. The samples were then partially cleared by treating them with 4% w/v SDS in 1×PBS for 2 minutes at room temperature. After this treatment, samples were washed three times with 1×PBS for 5 minutes, and then immersed in 70% ethanol and stored at 4 °C for at least 18 hours.

Tissue samples were then stained and cleared following the protocols described above. Tissue samples were measured using 2-color MERFISH as described above. Four cryosections were imaged in a single MERFISH experiment on the high-throughput imaging platform described above.

While several embodiments of the present invention have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present invention. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used. Those skilled in the art will recognize, or be able to ascertain using no more than routine experimentation, many equivalents to the specific embodiments of the invention described herein. It is, therefore, to be understood that the foregoing embodiments are presented by way of example only and that, within the scope of the appended claims and equivalents thereto, the invention may be practiced otherwise than as specifically described and claimed. The present invention is directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present invention.

In cases where the present specification and a document incorporated by reference include conflicting and/or inconsistent disclosure, the present specification shall control. If two or more documents incorporated by reference include conflicting and/or inconsistent disclosure with respect to each other, then the document having the later effective date shall control.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

When the word "about" is used herein in reference to a number, it should be understood that still another embodiment of the invention includes that number not modified by the presence of the word "about."

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

The present disclosure also includes the following items.

1. A method, comprising:

> exposing a sample to a plurality of nucleic acid probes;
> polymerizing a gel within the sample;
> anchoring a target to the gel;
> clearing non-targets from the sample; and
> determining the targets within the gel by determining binding of the nucleic acid probes by imaging.

2. The method of item 1, wherein the target is a nucleic acid.

3. The method of any one of items 1 or 2, wherein the target comprises RNA.

4. The method of any one of items 1 or 2, wherein the target comprises DNA.

5. The method of any one of items 1-4, wherein anchoring the target to the gel comprises anchoring the target to a nucleic acid probe and covalently bonding the nucleic acid probe to the gel.

6. The method of any one of items 1-5, wherein anchoring the target to the gel comprises anchoring the target to a nucleic acid probe and noncovalently bonding the nucleic acid probe to the gel.

7. The method of any one of items 1-6, wherein anchoring the target to the gel comprises anchoring the target to the gel via hybridization to the nucleic acid probes.

8. The method of any one of items 1-7, wherein anchoring the target to the gel comprises anchoring the target to the gel via covalently bonding the target to the nucleic acid probes.

9. The method of any one of items 1-8, wherein anchoring the target to the gel comprises anchoring the target to the gel by physically entangling the target with the gel.

10. The method of any one of items 1-9, wherein anchoring the target to the gel comprises covalently binding the target directly to the gel.

11. The method of any one of items 1-10, wherein anchoring the target to the gel comprises noncovalently binding the target directly to the gel.

12. The method of any one of items 1-11, wherein anchoring the target to the gel occurs during polymerizing the gel within the sample.

13. The method of item 12, wherein the target is anchored to a gel precursor prior to polymerizing the gel precursor to form the gel within the sample.

14. The method of any one of items 1-13, wherein anchoring the target to the gel occurs after polymerizing the gel within the sample.

15. The method of item 14, wherein after polymerizing the gel within the sample, the gel and/or the target is modified to anchor the target to the gel.

16. The method of any one of items 1-15, wherein clearing non-targets from the sample occurs after anchoring the target to the gel.

17. The method of any one of items 1-16, wherein exposing the sample to the plurality of nucleic acid probes occurs prior to clearing non-targets from the sample.

18. The method of any one of items 1-17, wherein exposing the sample to the plurality of nucleic acid probes occurs after clearing non-targets from the sample.

19. The method of any one of items 1-18, wherein the non-targets include proteins.

20. The method of any one of items 1-19, wherein the non-targets include lipids.

21. The method of any one of items 1-20, wherein the non-targets include nucleic acid

22. The method of item 21, wherein the non-targets include DNA.

23. The method of any one of items 21 or 22, wherein the non-targets include RNA.

24. The method of any one of items 1-23, wherein the non-targets include a carbohydrate.

25. The method of any one of items 1-24, wherein the non-targets include extracellular matrix.

26. The method of any one of items 1-25, wherein during imaging, the gel has not expanded by more than 3x.

27. The method of any one of items 1-26, wherein during imaging, the gel has not expanded by more than 1.5x.

28. The method of any one of items 1-27, wherein the plurality of nucleic acid probes comprises smFISH probes.

29. The method of any one of items 1-28, wherein the plurality of nucleic acid probes comprises MERFISH probes.

30. The method of any one of items 1-29, wherein the plurality of nucleic acid probes comprises anchor probes able to polymerize with the gel.

31. The method of any one of items 1-29, wherein the plurality of nucleic acid probes comprises anchor probes able to associate with the target and polymerize into the gel.

32. The method of any one of items 30 or 31, wherein at least some of the anchor probes comprises a poly-dT portion.

33. The method of any one of items 30-32, wherein at least some of the anchor probes comprises alternating dT and locked dT portions.

34. The method of item 33, wherein at least some of the anchor probes comprises a 15-nt sequence of alternating dT and locked dT portions.

35. The method of any one of items 30-34, wherein at least some of the anchor probes comprises an acrydite portion able to polymerize with the gel.

36. The method of item 35, wherein the acrydite portion is bound to the 5' end.

37. The method of item 35, wherein the acrydite portion is bound to the 3' end.

38. The method of item 35, wherein the acrydite portion is bound to an internal base.

39. The method of any one of items 1-38, wherein the gel comprises polyacrylamide.

40. The method of any one of items 1-39, wherein the gel comprises agarose.

41. The method of any one of items 1-40, wherein clearing non-targets from the sample comprises exposing the gel to a proteinase.

42. The method of item 41, wherein the proteinase comprises proteinase K.

43. The method of any one of items 1-42, wherein clearing non-targets from the sample comprises exposing the gel to guanidine HCl.

44. The method of any one of items 1-43, wherein clearing non-targets from the sample comprises exposing the gel to Triton X-100 (polyethylene glycol p-(1,1,3,3-tetramethylbutyl)-phenyl ether).

45. The method of any one of items 1-44, wherein clearing non-targets from the sample comprises exposing the gel to sodium dodecyl sulfate.

46. The method of any one of items 1-45, wherein clearing non-targets from the sample comprises exposing the gel to ethylenediaminetetraacetic acid.

47. The method of any one of items 1-46, wherein clearing non-targets from the sample comprises removing proteins and/or lipids from the sample.

48. The method of any one of items 1-47, wherein clearing non-targets from the sample comprises degrading proteins and/or lipids from the sample.

49. The method of any one of items 1-48, wherein clearing non-targets from the sample comprises removing DNA from the sample.

50. The method of item 49, wherein removing DNA from the sample comprises exposing the sample to a DNAse.

51. The method of any one of items 1-50, wherein the nucleic acid probes comprise a first portion comprising a target sequence and a second portion comprising one or more read sequences.

52. The method of item 51, further comprising determining read sequences based on determining binding of the read sequences bound to the gel.

53. The method of any one of items 51 or 52, comprising creating codewords or barcodes based on determination of the read sequences within the gel.

54. The method of any one of items 51-53, wherein the read sequences are taken from a set of orthogonal sequences, which have a homology of less than 15 basepairs with one another and with the nucleic acid species in a sample.

55. The method of any one of items 1-54, wherein the sample comprises a cell.

56. The method of any one of items 1-55, wherein the sample comprises a tissue.

57. The method of any one of items 1-56, comprising imaging using fluorescence microscopy.

58. The method of any one of items 1-57, comprising imaging using epi-fluorescence microscopy, total-internal-reflectance microscopy, highly-inclined thin-illumination (HILO) microscopy, light-sheet microscopy, scanning confocal microscopy, scanning line confocal microscopy, or spinning disk confocal microscopy.

59. The method of any one of items 1-58, comprising imaging using multiplexed fluorescence in situ hybridization.

60. The method of any one of items 1-59, comprising imaging using multiplexed error robust fluorescence in situ hybridization (MERFISH).

61. The method of any one of items 1-60, comprising imaging using multiple rounds of fluorescence in situ hybridization.

62. The method of any one of items 1-61, comprising imaging using multiple rounds of fluorescence in situ hybridization wherein, in each round, one or more different nucleic acid probes, each conjugated to a spectrally distinct fluorescent dye are used to readout out multiple readout sequences simultaneously.

63. The method of any one of items 1-62, comprising imaging at a resolution better than 500 nm.

64. The method of any one of items 1-63, comprising imaging using a technique selected from the group consisting of STORM, PALM, FPALM, STED, SIM, RESOLFT, SOFI or SPDM.

65. A method, comprising:

exposing a sample to a plurality of nucleic acid probes;
polymerizing a gel within the sample;
anchoring a target to the gel;
reducing background fluorescence within the sample; and
imaging the nucleic acid probes.

66. The method of item 65, wherein during imaging, the gel has not expanded by more than 3x.

67. The method of any one of items 65 or 66, wherein during imaging, the gel has not expanded by more than 1.5x.

68. The method of any one of items 65-67, wherein the plurality of nucleic acid probes comprises smFISH probes.

69. The method of any one of items 65-68, wherein the plurality of nucleic acid probes comprises MERFISH probes.

70. The method of any one of items 65-69, wherein the plurality of nucleic acid probes comprises anchor probes able to polymerize with the gel.

71. The method of item 70, wherein at least some of the anchor probes comprises a poly-dT portion.

72. The method of item 71, wherein at least some of the anchor probes comprises alternating dT and locked dT portions.

73. The method of item 72, wherein at least some of the anchor probes comprises a 15-nt sequence of alternating dT and locked dT portions.

74. The method of any one of items 70-73, wherein at least some of the anchor probes comprises an acrydite portion able to polymerize with the gel.

75. The method of item 74, wherein the acrydite portion is bound to the 5' end.

76. The method of item 74, wherein the acrydite portion is bound to the 3' end.

77. The method of item 74, wherein the acrydite portion is bound to an internal base.

78. The method of any one of items 65-77, wherein the gel comprises polyacrylamide.

79. The method of any one of items 65-78, wherein the gel comprises agarose.

80. The method of any one of items 65-79, wherein reducing background fluorescence comprises clearing cellular components.

81. The method of any one of items 65-80, wherein reducing background fluorescence comprises clearing components that quench fluorescent molecules.

82. The method of any one of items 65-81, wherein reducing background fluorescence comprises clearing autofluorescent components.

83. The method of item 82, wherein clearing autofluorescent components comprises reacting the autofluorescent components.

84. The method of any one of items 82 or 83, wherein reacting the autofluorescent components comprises exposing the gel to a proteinase.

85. The method of item 84, wherein the proteinase comprises proteinase K.

86. The method of any one of items 82-85, wherein reacting the autofluorescent components comprises exposing the gel to guanidine HCl.

87. The method of any one of items 82-86, wherein reacting the autofluorescent components comprises exposing the gel to Triton X-100 (polyethylene glycol p-(1,1,3,3-tetramethylbutyl)-phenyl ether).

88. The method of any one of items 82-87, wherein reacting the autofluorescent components comprises exposing the gel to sodium dodecyl sulfate.

89. The method of any one of items 82-88, wherein reacting the autofluorescent components comprises exposing the gel to ethylenediaminetetraacetic acid.

90. The method of any one of items 82-89, wherein reacting the autofluorescent components comprises removing proteins and/or lipids from the sample.

91. The method of any one of items 82-90, wherein reacting the autofluorescent components comprises degrading proteins and/or lipids from the sample.

92. The method of any one of items 65-91, wherein the nucleic acid probes comprise a first portion comprising a target sequence and a second portion comprising one or more read sequences.

93. The method of item 92, further comprising determining read sequences based on determining binding of the read sequences bound to the gel.

94. The method of any one of items 92 or 93, comprising creating codewords based on determination of the read sequences within the gel.

95. The method of any one of items 92-94, wherein the read sequences are taken from a set of orthogonal sequences, which have a homology of less than 15 basepairs with one another and with the nucleic acid species in a sample.

96. The method of any one of items 65-95, wherein the sample comprises a cell.

97. The method of any one of items 65-96, wherein the sample comprises a tissue.

98. The method of any one of items 65-97, comprising imaging using fluorescence microscopy.

99. The method of any one of items 65-98, comprising imaging using epi-fluorescence microscopy, total-internal-reflectance microscopy, highly-inclined thin-illumination (HILO) microscopy, light-sheet microscopy, scanning confocal microscopy, scanning line confocal microscopy, spinning disk confocal microscopy, or other comparable conventional microscopy techniques.

100. The method of any one of items 65-99, comprising imaging using multiplexed fluorescence in situ hybridization.

101. The method of any one of items 65-100, comprising imaging using multiplexed error robust fluorescence in situ hybridization (MERFISH).

102. The method of any one of items 65-101, comprising imaging using multiple rounds of fluorescence in situ hybridization.

103. The method of any one of items 65-102, comprising imaging at a resolution better than 500 nm.

104. The method of any one of items 65-103, comprising imaging using a technique selected from the group consisting of STORM, PALM, FPALM, STED, SIM, RESOLFT, SOFI or SPDM.

105. The method of any one of items 65-104, wherein anchoring the target to the gel comprises anchoring the target to a nucleic acid probe and covalently bonding the nucleic acid probe to the gel.

106. The method of any one of items 65-105, wherein anchoring the target to the gel comprises anchoring the target to a nucleic acid probe and noncovalently bonding the nucleic acid probe to the gel.

107. The method of any one of items 65-106, wherein anchoring the target to the gel comprises anchoring the target to the gel via hybridization to the nucleic acid probes.

108. The method of any one of items 65-107, wherein anchoring the target to the gel comprises anchoring the target to the gel via covalently bonding the target to the nucleic acid probes.

109. The method of any one of items 65-108, wherein anchoring the target to the gel comprises anchoring the target to the gel by physically entangling the target with the gel.

110. The method of any one of items 65-109, wherein anchoring the target to the gel comprises covalently binding the target directly to the gel.

111. The method of any one of items 65-110, wherein anchoring the target to the gel comprises noncovalently binding the target directly to the gel.

112. The method of any one of items 65-111, wherein anchoring the target to the gel occurs during polymerizing the gel within the sample.

113. The method of item 112, wherein the target is anchored to a gel precursor prior to polymerizing the gel precursor to form the gel within the sample.

114. The method of any one of items 65-113, wherein anchoring the target to the gel occurs after polymerizing the gel within the sample.

115. The method of item 114, wherein after polymerizing the gel within the sample, the gel is modified to anchor the target to the gel.

116. The method of any one of items 65-115, wherein reducing background fluorescence occurs after anchoring the target to the gel.

117. The method of any one of items 65-116, wherein exposing the sample to the plurality of nucleic acid probes occurs prior to reducing background fluorescence.

118. The method of any one of items 65-117, wherein exposing the sample to the plurality of nucleic acid probes occurs after reducing background fluorescence.

119. A method, comprising:

    exposing a sample to a plurality of MERFISH nucleic acid probes;
    exposing a sample to a plurality of anchor nucleic acid probes;
    embedding at least a portion of the sample within a polyacrylamide gel;
    immobilizing at least some of the anchor nucleic acid probes to the polyacrylamide gel;
    clearing proteins and/or lipids and/or DNA and/or extracellular matrix and/or RNA molecules from the sample; and
    determining binding of the MERFISH nucleic acid probes by imaging the polyacrylamide gel.

120. The method of item 119, wherein the polyacrylamide gel comprises anchor probes incorporated within the polyacrylamide gel.

121. The method of any one of items 119 or 120, wherein clearing proteins and/or lipids from the sample comprises removing proteins and/or lipids from the sample.

122. The method of any one of items 119-121, wherein clearing proteins and/or lipids from the sample comprises degrading proteins and/or lipids from the sample.

123. The method of any one of items 119-122, wherein clearing removing DNA and/or RNA and/or extracellular matrix from the sample.

124. The method of any one of items 119-123, wherein clearing comprises degrading DNA and/or RNA and/or extracellular matrix.

125. The method of any one of items 119-124, wherein the nucleic acid probes comprise a first portion comprising a target sequence and a second portion comprising one or more read sequences.

126. The method of item 125, further comprising determining read sequences based on determining binding of the read sequences bound to target RNAs.

127. The method of any one of items 125 or 126, comprising creating codewords or barcodes based on determination of the read sequences within the gel.

128. The method of any one of items 125-127, wherein the read sequences are taken from a set of orthogonal sequences, which have a homology of less than 15 basepairs with one another and with the nucleic acid species in a sample.

129. The method of any one of items 119-128, wherein at least some of the anchor probes comprises a poly-dT portion.

130. The method of item 129, wherein at least some of the anchor probes comprises alternating dT and locked dT portions.

131. The method of item 130, wherein at least some of the anchor probes comprises a 15-nt sequence of alternating dT and locked dT portions.

132. The method of any one of items 119-131, wherein at least some of the anchor probes comprises an acrydite portion able to polymerize with the gel.

133. The method of item 132, wherein the acrydite portion is bound to the 5' end.

134. The method of item 132, wherein the acrydite portion is bound to the 3' end.

135. The method of item 132, wherein the acrydite portion is bound to an internal base.

136. The method of any one of items 119-135, wherein clearing comprises exposing the gel to a proteinase.

137. The method of item 136, wherein the proteinase comprises proteinase K.

138. The method of any one of items 119-137, wherein clearing comprises exposing the gel to guanidine HCl.

139. The method of any one of items 119-138, wherein clearing comprises exposing the gel to Triton X-100 (polyethylene glycol p-(1,1,3,3-tetramethylbutyl)-phenyl ether).

140. The method of any one of items 119-139, wherein clearing comprises exposing the gel to sodium dodecyl sulfate.

141. The method of any one of items 119-140, wherein clearing comprises exposing the gel to ethylenediaminetetraacetic acid.

142. The method of any one of items 119-141, wherein clearing comprises removing proteins and/or lipids from the sample.

143. The method of any one of items 119-142, wherein clearing comprises degrading proteins and/or lipids from the sample.

144. The method of any one of items 119-143, wherein clearing non-targets from the sample comprises removing DNA from the sample.

145. The method of item 144, wherein removing DNA from the sample comprises exposing the sample to a DNAse.

146. The method of any one of items 119-145, wherein anchoring the target to the gel occurs during polymerizing the gel within the sample.

147. The method of item 146, wherein the target is anchored to a gel precursor prior to polymerizing the gel precursor to form the gel within the sample.

148. The method of any one of items 119-147, wherein anchoring the target to the gel occurs after polymerizing the gel within the sample.

149. The method of any one of items 119-148, wherein the acts are performed in the order recited.

150. The method of any one of items 119-149, wherein clearing occurs prior to exposing the sample to the plurality of anchor nucleic acid probes.

151. A method, comprising:

embedding at least a portion of a sample within a matrix;
immobilizing targets to the matrix;
clearing non-targets from the matrix; and
imaging the targets within the matrix.

152. The method of item 151, wherein the matrix comprises a polymer.

153. The method of any one of items 151 or 152, wherein the matrix comprises a gel.

154. The method of any one of items 151-153, wherein the target comprises nucleic acids.

155. The method of any one of items 151-154, wherein the target comprises proteins.

156. The method of any one of items 151-155, wherein immobilizing targets to the matrix comprises incorporating an anchor probe to the matrix, wherein the anchor probe specifically binds the targets.

157. The method of item 156, wherein the anchor probe comprises a nucleic acid able to specifically bind the targets.

158. The method of any one of items 156 or 157, wherein the anchor probe comprises an antibody able to specifically bind the targets.

159. The method of any one of items 156-158, wherein the anchor probe comprises a chemical crosslinker capable of covalently or non-covalently binding the specific targets and the matrix.

160. The method of any one of items 151-159, wherein the target molecules are anchored to the matrix via physical entanglement within the matrix.

161. The method of any one of items 151-160, wherein clearing non-targets comprises removing the non-targets from the matrix.

162. The method of any one of items 151-161, wherein clearing non-targets comprises degrading the non-targets.

163. The method of any one of items 151-162, wherein clearing non-targets comprises exposing the sample to an enzyme able to degrade a protein.

164. The method of any one of items 151-163, wherein clearing non-targets comprises exposing the sample to a detergent.

165. The method of any one of items 151-164, wherein clearing non-targets comprises exposing the sample to an enzyme able to degrade DNA.

166. The method of any one of items 151-165, wherein clearing non-targets comprises exposing the sample to an enzyme able to degrade RNA.

167. The method of any one of items 151-166, wherein clearing non-targets comprises exposing the sample to an enzyme able to degrade sugars or sugar-modified biomolecules.

168. The method of any one of items 151-167, wherein imaging the targets comprises imaging using optical microscopy.

169. The method of any one of items 151-168, wherein imaging the targets comprises imaging using fluorescence microscopy.

**Claims**

1. A method, comprising:

   exposing a sample to a plurality of MERFISH nucleic acid probes comprising a first portion comprising a target sequence and second portion comprising one or more read sequences;
   exposing a sample to a plurality of anchor nucleic acid probes, wherein the anchor probes specifically hybridize to the nucleic acid targets;
   embedding at least a portion of the sample within a polyacrylamide gel;
   immobilizing at least some of the anchor nucleic acid probes to the polyacrylamide gel;
   clearing proteins and/or lipids and/or DNA and/or extracellular matrix and/or RNA molecules from the sample comprising removing proteins and/or lipids from the sample and/or removing DNA and/or RNA and/or extracellular matrix from the sample; and
   determining binding of the MERFISH nucleic acid probes to the nucleic acid targets by imaging the polyacrylamide gel.

2. The method of claim 1, wherein the polyacrylamide gel comprises the anchor probes incorporated within the polyacrylamide gel.

3. The method of claim 1, further comprising determining read sequences based on determining binding of the read sequences bound to target RNAs.

4. The method of any one of claims 1-3, comprising creating codewords or barcodes based on determination of the read sequences within the gel.

5. The method of any one of claims 1-4, wherein the read sequences are taken from a set of orthogonal sequences, which have a homology of less than 15 basepairs with one another and with the nucleic acid target in the sample.

6. The method of any one of claims 1-5, wherein at least some of the anchor probes comprises a poly-dT portion.

7. The method of claim 6, wherein at least some of the anchor probes comprises alternating dT and locked dT portions.

8. The method of any one of claims 1-7, wherein at least some of the anchor probes comprises an acrydite portion able to polymerize with the gel.

9. The method of any one of claims 1-8, wherein clearing comprises exposing the gel to a proteinase, guanidine HCl, Triton X-100 (polyethylene glycol p-(I,I,3,3-tetramethylbutyl)-phenyl ether), sodium dodecyl sulfate, ethylenediaminetetraacetic acid.

10. The method of any one of claims 1-9, wherein anchoring the nucleic acid target to the gel occurs during polymerizing the gel within the sample.

11. The method of any one of claims 1-9, wherein anchoring the nucleic acid target to the gel occurs after polymerizing the gel within the sample.

12. The method of claim 1, wherein immobilizing nucleic acid targets to the gel comprises incorporating the anchor probe to the gel, wherein the anchor probe specifically binds the ta rgets.

13. The method of claim 1, wherein the anchor probe comprises a chemical crosslinker capable of covalently or non-covalently binding the specific targets and the matrix.

14. The method of claim 1, wherein imaging the targets comprises imaging using optical microscopy or fluorescence microscopy.

15. The method of claim 14, comprising imaging using epi-fluorescence microscopy, total-internal-reflectance microscopy, highly-inclined thin-illumination (HILO) microscopy, light-sheet microscopy, scanning confocal microscopy, scanning line confocal microscopy, spinning disk confocal microscopy, or other comparable conventional microscopy techniques.

FIG. 1A

FIG. 1B

FIG. 1C

FIG. 2A

FIG. 2B

FIG. 2C

FIG. 2D

FIG. 3A

FIG. 3B

FIG. 3C

FIG. 3D

FIG. 3E

FIG. 4A

FIG. 4B

FIG. 4C

FIG. 4D

FIG. 4E

FIG. 4F

FIG. 4G

FIG. 5A

FIG. 5B

FIG. 5C

FIG. 6

Uncleared     Cleared     Cleared

20 µm

10x contrast

FIG. 7A

FIG. 7B

Uncleared      Cleared      Cleared

20 μm            10x contrast

## FIG. 7C

## FIG. 7D

FIG. 8A

FIG. 8B

FIG. 9A

FIG. 9B

FIG. 10

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62419033, Zhuang
- WO 2016018960 A
- WO 2016018963 A
- US 7838302 B, Zhuang
- US 8564792 B, Zhuang
- WO 2013090360 A, Zhuang
- US 61979436
- US 2015042556 W, Zhuang
- US 2015042559 W, Zhuang

**Non-patent literature cited in the description**

- **CHEN, K.H. et al.** Spatially resolved, highly multiplexed RNA profiling in single cells. *Science,* vol. 348 (6233), aaa6090
- **MOFFITT, J.R. et al.** High-throughput single-cell gene-expression profiling with multiplexed error-robust fluorescence in site hybridization. *Proc. Natl. Acad. Sci. USA,* vol. 113 (39), 11046-11051
- **MOFFITT, J.R. et al.** RNA Imaging with Multiplexed Error-Robust Fluorescence In Situ Hybridization (MERFISH). *Methods Enzymol.,* 2016, vol. 572, 1-49